# EUROPEAN PATENT SPECIFICATION

(11) **EP 4 255 311 B1**
(45) Date of publication and mention of the grant of the patent: **02.10.2024**
(21) Application number: 22737667.0
(22) Date of filing: 06.07.2022
(51) Int. Cl.: A61B 8/08, A61B 8/00

(54) **ROBOTIZED IMAGING SYSTEM**
ROBOTISIERTES ABBILDUNGSSYSTEM
SYSTÈME D'IMAGERIE ROBOTISÉ

(30) Priority: 06.07.2021 NL 2028640
(43) Date of publication of application: 11.10.2023
(73) Proprietor: Corbotics B.V., 2629 JD Delft (NL)
(72) Inventor: RONNER, Eelko, 2629JD Delft (NL); RIEZEBOS, Robert, 2629JD Delft (NL); TRIP, Kenrick, 2629JD Delft (NL); BALLAST, Fabian, 2629JD Delft (NL); ZHANG, Ying, 2629JD Delft (NL); WILLEMS VAN DIJK, Janne, 2629JD Delft (NL); VAN STIJN, Nick, 2629JD Delft (NL); KLAZINGA, Rembrandt, 2629JD Delft (NL); DHAREENDRA PATIL, Sandeep, 2629JD Delft (NL)
(74) Representative: De Vries & Metman
(86) International application number: PCT/NL2022/050388
(87) International publication number: WO 2023/282743

(56) References cited:
- WO-A1-2020/188064
- US-A1- 2019 328 500
- US-A1- 2020 194 117

## Description

### Technical field

The embodiments in this disclosure relate to robotized imaging systems.

### Background

Cardiovascular diseases are the number one cause of mortality worldwide. An essential means for identification of such diseases is an ultrasonic imaging system known as echocardiography, an acoustic imaging technique for generating echocardiograms. More generally, sonography or ultrasonography is an important diagnostic imaging technique for many medical procedures, wherein ultrasound waves are used to create an image of internal body structures such as tendons, muscles, joints, blood vessels, and internal organs. Making and interpreting sonograms is a skilled labour. For example, ultrasound images in 14 up to 37 different view directions are captured during a full examination of the heart by a medical specialist, wherein each of these views is associated with different positions and orientations of the probe relative to the body.

During this process, a medical specialist will manually position an ultrasound probe at a predetermined position such that the targeted view direction is shown on a display. When the display shows the correct image, a short video will be recorded showing the motion of the heart. This process will be repeated for each of the required views. In order to minimize interference effects in the images due to objects and/or tissue close to the heart, such as (parts of) the rib cage and lung tissue, the pose of the patient (lying on the back or side in a certain pose) will be changed depending on the view direction that is recorded.

The result of the examination is a set of ultrasound images associated with different view directions which may be analyzed by the medical specialist. Due to a shortage of skilled medical specialists however, there is a high threshold and/or relatively long waiting time for patient to get a scan, increasing the risk of hospitalization and sometimes severe medical consequences for a patient.

To relieve pressure on medical specialists there is a need to automate medical imaging schemes, such an echocardiography scheme, for routine patients so that human interference is not needed or at least minimized. Robotized imaging systems in which a robot arm comprising an imaging probe is controlled to produce images of a patient are known. For example, WO2019/174953 describes a robotized imaging system including a robotic arm that can be controlled to position an imaging probe at a predetermined position at a body part of a patient lying on the back (in supine position) one a bed. A robotic system is configured to control the robotic arm based on a neural network system, which is trained to receive an ultrasound image generated by the probe and to produce a control signal for the robotic system to move the probe in a direction of a target position. The neural network system includes different functional neural networks which need to be trained based on data, which is for a large part related to synthetic data produced on the basis of a phantom and for another part manually labelled clinical data. The data is subsequently used to train a neural network to position the probe at a target position that is associated with a predetermined clinical property. However, this system is not adapted to autonomously produce a medical examination of the heart of a patient in multiple view directions to produce a series of multi-view echocardiograms that is similar or substantially similar to those produced by a skilled sonographer. US2020/194117 describes a remotely controlled robotic ultrasound system wherein a machine learning model is used to examine conventional camera images of the patient to determine areas of the patient that should not be contacted with the ultrasound probe. Also this system is not adapted to autonomously produce a medical examination of the heart of a patient in multiple view directions to produce a series of multi-view echocardiograms

A further problem relates to the fact that training a neural network for a large number of views as is required for a full examination by a sonographer requires large amounts of labelled data, preferably clinical data of a certain quality. Such data is not available and manually producing such amounts of data is difficult and expensive.

Hence, from the above, it follows that there is a need in the art for improved methods and systems for robotized ultrasound imaging system, such as an ultrasound echocardiographic system, in particular there is a need in methods and systems for robotized ultrasound imaging configured to autonomously determine a full echocardiographic set of images for different views that is of the same quality as a set of images determined by a sonographer using a conventional echocardiographic system.

### Summary

As will be appreciated by one skilled in the art, aspects of the present invention are embodied as a system.

Aspects of the present disclosure may take the form of an entirely hardware embodiment, an entirely software embodiment (including firmware, resident software, micro-code, etc.) or an embodiment combining software and hardware aspects that may all generally be referred to herein as a "circuit," "module" or "system." Functions described in this disclosure may be implemented as an algorithm executed by a microprocessor of a computer. Furthermore, aspects of the present disclosure may take the form of a computer program product embodied in one or more computer readable medium(s) having computer readable program code embodied, e.g., stored, thereon.

Any combination of one or more computer readable medium(s) may be utilized. The computer readable medium may be a computer readable signal medium or a computer readable storage medium. A computer readable storage medium may be, for example, but not limited to, an electronic, magnetic, optical, electromagnetic, infrared, or semiconductor system, apparatus, or device, or any suitable combination of the foregoing. More specific examples (a non-exhaustive list) of the computer readable storage medium would include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of this document, a computer readable storage medium may be any tangible medium that can contain, or store a program for use by or in connection with an instruction execution system, apparatus, or device.

A computer readable signal medium may include a propagated data signal with computer readable program code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

Program code embodied on a computer readable medium may be transmitted using any appropriate medium, including but not limited to wireless, wireline, optical fiber, cable, RF, etc., or any suitable combination of the foregoing. Computer program code for carrying out operations for aspects of the present invention may be written in any combination of one or more programming languages, including an object-oriented programming language such as Java(TM), Smalltalk, C++ or the like and conventional procedural programming languages, such as the "C" programming language or similar programming languages. The program code may execute entirely on the user's computer, partly on the user's computer, as a stand-alone software package, partly on the user's computer and partly on a remote computer, or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present disclosure are described below with reference to flowchart illustrations and/or block diagrams of methods, apparatus (systems), and computer program products according to the disclosure.

It will be understood that each block of the flowchart illustrations and/or block diagrams, and combinations of blocks in the flowchart illustrations and/or block diagrams, can be implemented by computer program instructions. These computer program instructions may be provided to a processor, in particular a microprocessor or central processing unit (CPU) or a graphical processing unit (GPU), of a general purpose computer, special purpose computer, or other programmable data processing apparatus to produce a machine, such that the instructions, which execute via the processor of the computer, other programmable data processing apparatus, or other devices create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks. Additionally, the Instructions may be executed by any type of processors, including but not limited to one or more digital signal processors (DSPs), general purpose microprocessors, application specific integrated circuits (ASICs), field programmable logic arrays (FP- GAs), or other integrated or discrete logic circuitry.

The flowchart and block diagrams in the figures illustrate the architecture, functionality, and operation of possible implementations of systems, methods and computer program products according to the present disclosure.

In this regard, each block in the flowchart or block diagrams may represent a module, segment, or portion of code, which comprises one or more executable instructions for implementing the specified logical function(s). It should also be noted that, in some alternative implementations, the functions noted in the blocks may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustrations, and combinations of blocks in the block diagrams and/or flowchart illustrations, can be implemented by special purpose hardware-based systems that perform the specified functions or acts, or combinations of special purpose hardware and computer instructions.

It is an aim of the embodiments in this application to provide systems for robotized medical imaging, such as ultrasound echography, allowing autonomous determination of a full set of images of different target views.

In an aspect, an embodiment relates to a robotized ultrasound imaging system according to claim 1.

In an embodiment, the data generating module may be further configured to determine a plurality of training positions for the probe within a predetermined data collection area defined around a target position; move the probe to each of the training positions and capture one or more images for each of the training positions; associating the one or more images associated with a training position with direction information, the direction information including coordinates of a vector pointing from the training position to the target position; and, storing the one or more images and associated direction information as clinical training data on a storage medium.

In an embodiment, the system may further comprise a training module configured to: provide an image associated with a training position to the input of the machine learning algorithm, the image being associated with direction information defining a target direction, preferably a target vector, pointing from the training position towards a target position; receiving estimated direction information, preferably an estimated vector, from the output of the machine learning algorithm and evaluating the estimated direction information based on the target vector and a loss function; adjust training parameters of the machine learning algorithm based on the evaluation of the estimated direction information; and, repeating the process of providing an image to the input of the machine learning algorithm, evaluating the output of the deep neural network and adjusting of the training parameters, until the evaluation indicates that the estimated vector substantially matches the vector.

In an embodiment, the execution module may be configured to: moving the probe to a first probe position against a body part of a patient and capturing one or more first images at the first probe position; providing the one or more first images to a deep neural network to determine estimated direction information associated with the first probe position, the estimated position information defining an estimated direction pointing from the first probe position towards an estimated probe position of the target view; storing first probe position and the estimate direction information in a memory; repeating the steps of moving the probe to a further probe position, determining estimated direction information for the further probe position using the trained deep neural network and storing the probe position and the associated estimated direction information until a set of probe positions and associated estimated direction information are collected; and, determining an optimal probe position of the target view based on the collected probe positions and estimated direction information and moving the probe to the optimal probe position to capture one or more images of the target view.

In an embodiment, the machine learning algorithm may be a deep neural network or a deep neural network system comprising a plurality of concatenated deep neural networks.

In an embodiment, the convolutional neural network or convolutional neural network system may be configured as a regression algorithm or a classification algorithm.

In an embodiment, the deep neural network or deep neural network system may comprise one or more convolutional neural networks for extracting features from ultrasound images captured by the probe; and/or, wherein the deep neural network or deep neural network system comprises a network of densely connected layers for transforming features associated with an ultrasound image captured at a probe position into estimated direction information, wherein the estimated direction information defines a direction pointing from the probe position towards an estimated probe position of the target position.

In an embodiment, the clinical training data further may include one or more features of one or more vital sign signals associated with the one or more captured ultrasound images, preferably the one or more features including features of an electrocardiogram ECG signal and/or features of a respiratory signal.

In an embodiment, the support structure may be configured to position the object in a prone position, the support structure comprising an opening or a recess exposing part of the object, preferably the chest of the object, to the probe, more preferably at least part of the robotic system being arranged under the support structure.

In contrast to known robotized echography systems, this embodiment, relies on the fact that a human object is positioned on a support structure that comprises an opening that provides a robotized probe to access the chest of a human object that lies on the support structure in a prone position. In certain viewing directions, scanning in prone position may be advantageous. For example, during echocardiographic imaging in the prone position the heart will less blurred by the lung tissue as is the case if a human object is lying on its back.

Thus, imaging a patient in the prone position will generate improved images, i.e. images with less distortions and/or interference due to lung tissue. Further, the opening in the support structure allows the accurately position and fixate a human object that needs to be scanned. Ultrasound images of internal tissue and/or organ of the human object in prone position that are generated by the data collector module may be used for generating training data for training the model. Training the algorithm based on the generated training data may result in a trained algorithm that can be used to make an accurately estimate of an an optimal probe position for determining ultrasonic images of a target view.

A further advantage of the system relates to the safety of the human object. In contrast to known robotized imaging systems, the human object is not positioned between the support structure, the bed, and the probe. Rather, the probe accesses the patient through an opening of the support structure so that the patient cannot get stuck between the probe and the bed, thus reducing the risk that malfunctioning of the robot may lead to situations in which the patient could be injured. Further embodiments and associated advantages of the system and methods executed by the system are described hereunder in more detail.

In an embodiment, the robotic system includes may include a robot arm connected to the imaging probe for moving the probe in translational and rotational directions; or a linear robotic system, preferably arranged under the support structure, wherein the robotic system includes a linear robotic system configured to move a probe stage comprising the imaging probe in translational directions and wherein the probe stage is configured to move the imaging probe in angular directions.

In an embodiment, the estimated directional information may be used by the robotic system to move the probe in one or more first directions (preferably the x,y directions) parallel to the plane of the support structure.

In an embodiment, a sensor signal of a pressure sensor or a force sensor associated with the probe may be used to move the probe in a second direction (preferably the z-direction) substantially perpendicular to the one or more first directions, preferably the sensor signal being used to press the probe with a constant force against the body part while the probe is moving in the one or more first directions.

In an embodiment, the probe stage may include a probe holder for holding the imaging probe, the probe holder including the pressure or force sensor, preferably the pressure or force sensor including a spring structure, such that when the probe is pressed against the body part of the object, the one spring structure will be compressed, wherein the compression of the spring structure represents a value of the force with which the probe is pressed against the body part.

In an embodiment, the direction information may include a set of translational coordinates for moving the imaging probe in a translational direction and a set of angular coordinates for moving the imaging probe in an angular direction.

In an embodiment, the remote controller may be shaped as a manual echography probe, the manual echography probe comprising one or more sensors.

In an embodiment, the sensors may be one or more accelerometer sensors, one or more optical navigation sensors and/or inclination sensors for translating a position of the manual echography probe into positional information for controlling the robotic system to move the imaging probe to a position according to the positional information.

In an aspect, not part of the invention, the disclosure relates to a robotized imaging system comprising: a robotic system configured to position an imaging probe against a body part of a patient positioned on a support structure and to move the probe over the body part; a computer connected to the robotic system, the computer comprising a computer readable storage medium having computer readable program code embodied therewith; and a processor, coupled to the computer readable storage medium, wherein responsive to executing the computer readable program code, the processor is configured to perform executable operations comprising: determine a plurality of training positions for the probe within a predetermined data collection area defined around a target position; move the probe to each of the training positions and capture one or more images for each of the training positions; associating the one or more images associated with a training position with direction information, the direction information defining a direction, for example a vector, pointing from the training position to the target position; and, storing the one or more images and associated direction information as clinical training data on a storage medium.

In an aspect, not part of the invention, the disclosure relates to a robotized imaging system comprising: a robotic system configured to position an imaging probe against a body part of a patient positioned on a support structure and to move the probe over the body part; a computer connected to the robotic system, the computer comprising a computer readable storage medium having computer readable program code embodied therewith; and a processor, coupled to the computer readable storage medium, wherein responsive to executing the computer readable program code, the processor is configured to perform executable operations comprising: provide an image associated with a training position to the input of the machine learning algorithm, the image being associated with direction information defining a direction, preferably a target vector, pointing from the training position towards a target position; receiving estimated direction information, preferably an estimated vector, from the output of the machine learning algorithm and evaluating the estimated direction information based on the target vector and a loss function; adjust training parameters of the machine learning algorithm based on the evaluation of the estimated direction information; and, repeating the process of providing an image to the input of the machine learning algorithm, evaluating the output of the deep neural network and adjusting of the training parameters, until the evaluation indicates that the estimated vector substantially matches the target vector.

In an aspect, not part of the invention, the disclosure relates to a robotized ultrasound imaging system comprising: a robotic system configured to position an imaging probe against a body part of a patient positioned on a support structure and to move the probe over the body part; a computer connected to the robotic system, the computer comprising a computer readable storage medium having computer readable program code embodied therewith; and a processor, coupled to the computer readable storage medium, wherein responsive to executing the computer readable program code, the processor is configured to perform executable operations comprising:
moving the probe to a first probe position against a body part of a patient and capturing one or more first images at the first probe position; providing the one or more first images to a deep neural network to determine estimated direction information associated with the first probe position, the estimated position information defining an estimated direction pointing from the first probe position towards an estimated probe position of the target view; storing first probe position and the estimate direction information in a memory; repeating the steps of moving the probe to a further probe position, determining estimated direction information for the further probe position using the trained deep neural network and storing the probe position and the associated estimated direction information until a set of probe positions and associated estimated direction information are collected; and,
determining an optimal probe position of the target view based on the collected probe positions and estimated direction information and moving the probe to the optimal probe position to capture one or more images of the target view.

The invention will be further illustrated with reference to the attached drawings, which schematically will show embodiments according to the invention. It will be understood that the invention is not in any way restricted to these specific embodiments.

### Brief description of the drawings

**Fig. 1** illustrates a robotized ultrasound echography system according to an embodiment;
**Fig. 2A** and **2B** depict a flow diagrams of methods for generating labelled training data for a robotized echography system;
**Fig. 3A** and **3B** illustrate pictures associated with the generation of training data;
**Fig. 4** illustrates a method for training a neural network;
**Fig. 5A** and **5B** depict a process for executing a process of autonomously generating a set of images of a body part of a patient;
**Fig. 6** depicts an example of an image that is used to determine a vector for moving the probe to a target location;
**Fig. 7A-7C** depict example of deep neural networks for controlling a robotized echography system;
**Fig. 8A** and **8B** depict an example of a robotized ultrasound echography system according to an embodiment;
**Fig. 9A-9C** depict various view of a rotatable probe for a robotized ultrasound echography system;
**Fig. 10A** and **10B** illustrates a robotized echography system according to an embodiment.

### Detailed description

The embodiments in this disclosure relate to robotized imaging systems, in particular medical robotized imaging system such as robotized ultrasound imaging systems. Such imaging systems are configured to determine images of internal tissue and/or organs an object, e.g. a human or an animal, for a medical procedure or physical examination. Such systems may for example include an ultrasound imaging system that is configured to determine ultrasound images, typically 2D slices, of the heart using an ultrasound imaging probe. The imaging probe produces sound waves, which enter the body and are reflected back by tissue and/or fluids, e.g. blood, which is detected by an ultrasound detector in the imaging probe. The type of the tissue and/or fluid determines the strength of the reflection, which is seen in the echo view as a brighter or darker spot. Typically, blood reflects very little, while muscle reflects more. This means that the structure of organs such as the heart (consisting largely of muscle and blood) can be imaged using sonography taking into account that tissue of other internal tissue, e.g. rib cage or lungs, may interfere with obtaining a clear picture of (part of) the heart in different view directions.

An ultrasound probe generates images in the form of a 2D slice of whatever it is aimed at, in a fan-like pattern. This fan can be seen as protruding from the head of the probe, slicing into the body. To look at different parts of a body part, e.g. the heart, a sonographer can manually rotate and/or laterally translate the probe around or over any axis. In echocardiography a number of predetermined "views" are defined. The axis of these view directions are fixed positions relative to the heart so that a sonographer can consistently use the different views to make a diagnosis and compare one set of images with another set of images. For instance, the so-called apical 4-chamber view (A4C) looks at the heart from below, through the apex, showing all four chambers of the heart. Another view is the parasternal long axis view (PLAX). To produce ultrasound images for this view, the ultrasound probe is placed perpendicular to the center of the chest (or sternum), and rotates it such that two chambers are visible: the left ventricle and the left atrium.

An ultrasound probe can capture images, also referred to as video frames, at a rate of more than 30 per second, thus allowing to make a video of a slice of the heart associated with a specific view direction. The result is a near-live video of the heart in motion, which allows echocardiographers diagnosing complex issues of the heart. Thus, an echography system may be configured to generate and process images of the heart according to predetermined standard view directions. Typically, images of 14 up to 37 different view directions may be captured during a full examination by a sonographer. In any case, at least five view directions are needed to reliably identify issues. A sonographer will position a probe at a predetermined position such that the targeted view direction is shown on a display. When the display shows the correct image, the sonographer will record a short video to show the motion of the heart. The sonographer will repeat this process for each of the required views, wherein for each view the sonographer will position the patient in a certain pose so in order to enable clear images. In order to minimize interference of internal tissue, such as (parts of) the rib cage and lung tissue, the sonographer will change the position of the patient (lying on the back or side in a certain pose) depending on the view direction that is recorded. The result of the examination by the sonographer is a set of ultrasound images associated with different views which can be analyzed by the cardiologist.

From the above it follows that a full echocardiography examination by a sonographer, which requires recording of the heart in multiple view directions in which the patient has to be repositioned various times, is laborious and physically exhausting, especially when examining multiple patients in one day. Furthermore, a lack of skilled sonographers causes long waiting times for patients. The embodiments in this application aim to solve at least part of the problems related to echography examination, in particular echocardiography examination, based on conventional echography systems. In particular, the embodiments relate to a robotized echography system, a robotized ultrasound imaging system, which allows autonomous echography examination in predetermined view directions that does not require human intervention or at least minimum human intervention.

While the examples in the figures are explained with reference to a robotized echography system, it is submitted that the embodiments in the application are not limited to robotized ultrasound imaging and may also be used with other types of robotized medical imaging systems for imaging internal tissue such as photon-acoustic imaging, tomographic imaging, radiographic imaging, x-ray imaging, magnetic resonance imaging, etc.

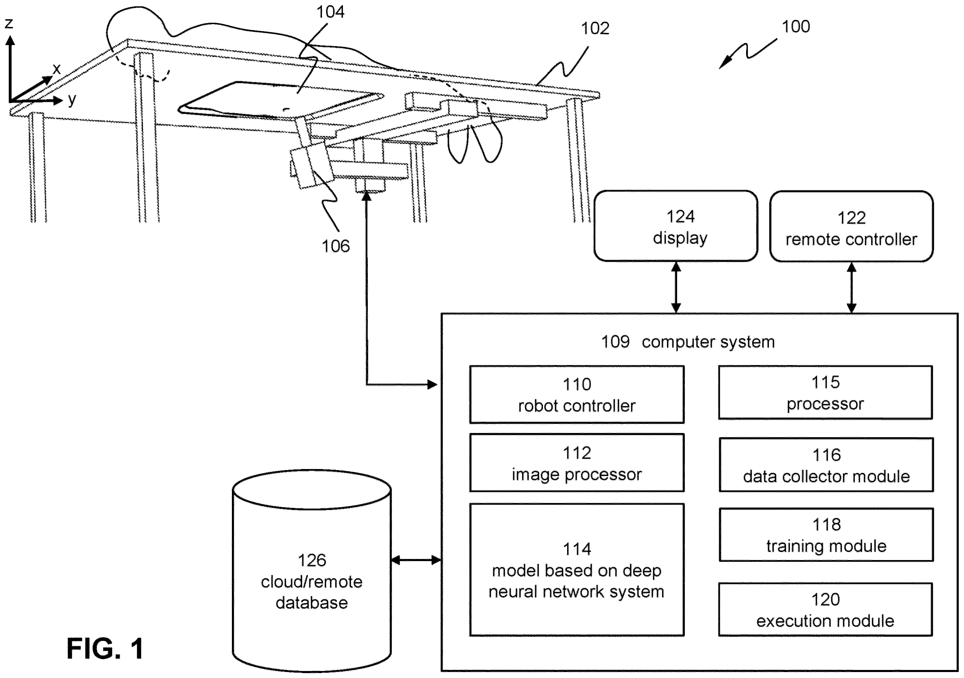

**Fig. 1** illustrates a robotized echography system **100** according to an embodiment of the invention. The system may include a support structure **102,** e.g. a bed, for positioning a patient in a pose for echography. In this particular embodiment, the support system may be configured so that a patient is positioned in a prone position. Further, the support system may include one or more openings **104** so that when a patient is lying in the prone position on the support system, at least part of the patient, e.g. the chest, is positioned over the one or more openings. This way, one or more body parts of the patient are exposed via the one or more openings in the positioning structure to an imaging probe which is connected to a robotic system **106.**

In the embodiment of **Fig. 1****,** the robotic system may be arranged at least partly under the support structure. The robotic system may be arranged to position and move the ultrasonic probe **108** relative to the support system in alignment with the body part of the patient. In particular, the robotic system may be arranged to move (translate) the probe in three axial directions relative to the support system. The position of the probe at a time instance may be defined based on suitable coordinate system, e.g. a cartesian coordinate system. The position of the probe may include coordinates (x,y,z) for translating the probe in three dimensions. Further, the position of the probe may also include an angular part (an angular direction) for rotating the probe in a certain pointing direction. For example, Euler angles (α, β, γ) may define the yaw, the pitch, and roll of the probe relative to the axis of the coordinate system. Thus, the movement of the probe, i.e. the (change in) position at each time instance may be described based on vectors (x,y,z,α,β,γ) including the translational and rotational coordinates.

The system further includes a computer system **109** comprising a processor **115** for executing different modules, e.g. controllers and/or modules associated with a model that are used to autonomously control the robotic system. For example, the computer system may include a robot controller **110** for controlling the robot system. The robot controller may be configured to control actuators and/or motors of the robot system to move and position the probe relative to the support system (and a patient positioned on the support system) based on information received from a machine learning model **114** or a remote controller **122.** Here, the machine learning model may include a system of one or more deep neural networks that are trained to move the probe from a current position towards an estimated target position. The machine learning model **114** may be used to autonomously control the robotic system. In that case, the processor of the computer system may set the system in an autonomous mode wherein based on ultrasound images produced by the ultrasound probe, the robot controller may send instructions to the robotic system to move the probe from a first position to a second position. Alternatively, the remote controller can be used to control the system in a human-controlled mode, wherein a person may manually control the robotic system.

The robotic system may further include sensors, e.g. force-, torque-, pressure-, position sensors, and/or accelerometers, for enabling control of the probe during scanning. For example, when the probe is positioned against the body of a patient, a force sensor may be used to exert a predetermined force against the body in the z-direction. For example, the robot controller may receive the sensor signal from the robotic system and use this signal as a force (or pressure) feedback signal. The force feedback may enable the probe controller to position and move the probe with a constant force and/or torque against the body of a patient lying on the support structure. Additionally, the force feedback will make sure that the pressure exerted onto a body part of the patient does not exceed a (threshold) value so that the positioning of the probe can be performed safely both when controlled by the machine learning model or by a person using the remote controller. This way the probe may be controlled to scan a patient by moving the probe over part of the body of a patient lying on the support structure, while capturing images, which may be processed by the computer to produce coordinates, e.g. in the form of a vector. At least part of the translational coordinates (e.g. the x and y direction) and the angular coordinates may be produced by the machine learning model. Another part of the translational coordinates (e.g. the z direction) may be controlled by the force feedback system as described above. In another embodiment, an optical navigation sensor may be used to accurately determine the position, e.g. translational motion, of the probe.

In the human-controlled mode, suitable user interfaces Uls, for example remote controller **122** and display **124,** connected to the system can be used by a sonographer to manually move the probe of the robotized echography system to a predetermined position in a certain view direction, while the probe produces images which are shown on the display. In an embodiment, the remote controller may be designed so that it can be used as a conventional hand-held probe which is configured detect translational and/or rotational movements. This way, the remote controller may have the look and feel of a conventional manual probe that are used in performing conventional sonography.

As will be described hereunder in greater detail, the system depicted in **Fig. 1** may be configured to autonomously create one or more set of images along predetermined view axes, e.g. the A4C and the PLAX view direction in case of echocardiography. The computer system may further include an image processor **112** for processing images, i.e. 2D pixelated pictures, which are generated when the probe is positioned relative to the chest of a patient to obtain images of the heart in predetermined view directions.

The robot controller may be controlled based on information that is determined by machine learning model **114,** e.g. a machine learning model such as a deep neural network or a system of deep neural networks. The model may be trained to control the robot system to move and orient the probe so that images of different target view can be determined. For each of the target views an optimal image of a target, e.g. an organ such as the heart, can be determined. This way, images of 2D slices of internal tissue or an organ, may be obtained in different view directions, for example, the view directions that a sonographer uses during an examination. To that end, the model may be configured to receive one or more images of internal tissue associated with a first probe position and produce based on the one or more images associated with a first view, an output signal defining information that can be used by the robot controller to move the probe towards a second probe position that can be used to capture images with a second view. This way, based on the information provided by the model, the robot controller can move the probe towards a probe position that is associated with the target view. In an embodiment, the output signal may include direction information, e.g. a vector or information for determining a vector, which can be used by the robot controller to move the probe from a first (current) probe position to a second probe position.

The machine learning model may be trained based on training data that may be stored on a storage medium **126.** Sufficient amounts of training data, preferably clinical training data associated with a heterogenous set of patients, is needed to train the model so that it can reliably control the probe to produce images for different predetermined view directions for patients of different size, gender and age. To enable autonomous scanning, a large data set is needed that does not only include images in the different view directions but also positional labels. Thus, to train the model, e.g. a deep neural network, to determine which way to move the probe when an image is provided to its input, each image may be labelled with direction information, e.g. a vector, defining a position relative to an optimal target position that is associated with a target view. This way, the model can learn to determine direction information, e.g. a vector pointing to a target position, based on echocardiographic images.

As no external datasets are available that have such properties, the computer system may be configured to generate labelled clinical training data sets itself. To that end, the computer may include a data collector module **116,** that is configured to generate labelled clinical data, i.e. capture and store images of a patient and associate these images with a position and orientation of the probe, e.g. in the format of a vector as described earlier. The computer system may further include a training module **118** configured to train the model based on the training data. Depending on the type of model, e.g. a deep neural network (DNN), such as a deep convolution neural network (CNN), may be trained iteratively wherein labelled images are input to the CNN and the output is evaluated by a loss function and weights of the DNN are adjusted based on gradient descent method, e.g. a stochastic gradient descent method. The computer system may further include an execution module **120** for executing the process of autonomously generating a set of images of a part of a patient, e.g. an organ such as the heart, in different view directions based on the trained model.

It is submitted that computer system in **Fig. 1** may be implemented in any form. For example, part or parts of the computer system may be implemented in the network, e.g. as a (remote) server application or cloud application. Further, the computer system may be a distributed system wherein some of the modules, e.g. the training module for training the neural network, may run on a remote computer.

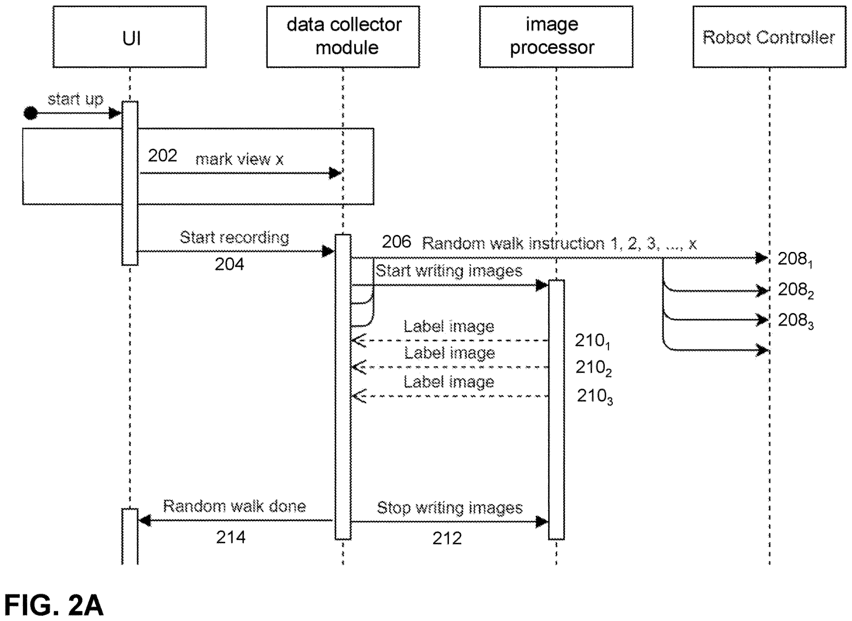

The data collector module may be configured to control the robotized echography system to generate training data. **Fig. 2A** depicts a flow diagram of a method for generating labelled training data for a robotized echography system.

In particular, the process may start with a user controlling a robotized echography system as e.g. described with reference to **Fig. 1****.** When a patient is lying on the support structure of the system, the user, e.g. a sonographer, may use a remote controller of the system to start a process of generating labelled training data. In a first step 202, the user may use the remote controller to control the robotic system and guide the probe to a target position that is associated with a target view along a certain viewing axis, while displaying images on a screen. Here, the target position may include a translational position (e.g. expressed in Cartesian coordinates) and an orientation (e.g. expressed in yaw, pitch, roll). Hence, the target position and associated target view of internal tissue, e.g. an organ, along a particular viewing direction may be determined by the sonographer using the remote controller of the system. For example, target views may relate to a set of views along a predetermined view axis, e.g. the A4C or the PLAX view direction in case of echocardiography. The sonographer may use the remote controller to mark that position as a target position (step **202**), which is associated with a target view of internal tissue, e.g. an organ such as the heart.

Once the target view and associated target position are defined, the data collector module may start data collection based on the target position of the probe (step **204**). The data collection process may include the data collector module providing a plurality of position instructions to the robot controller (step **206**). A position instruction may move the probe at training position relative to the target position (step **208**). When executing the position instructions, the training positions may be selected within a predetermined area around the target position. When the probe moves in a translational and/or angular direction, one or more images may be generated for the training positions of the probe. In an embodiment, the movements of the probe to the different training positions within the predetermined area may be based on a (pseudo)random value. In another embodiment, the movements may be based on a predetermined function.

Each of these images may be labelled with direction information (step **210₁**), wherein the direction information may define directions and, optionally, distances between training positions and the target position. Hence, the direction information may include vectors defined by a (current) position of the probe and a target position, The smaller the length of the vector, the closer the probe is to the desired target position. Thus, the direction information associated with an image indicates a direction from a current position towards an estimated target position. These labelled images may be stored as clinical training data on a storage medium of the system.

The process of executing a position instruction (step **208**_{**2**,**3**,...}), i.e. moving the probe to a training position and capturing an image, and associating, e.g. labelling, an image with the direction information (steps **210**_{**2**,**3**,...}) may be repeated many times so that a large collection of labelled images can be generated. Once a sufficient number of labelled images is generated, these images and associated direction information may be stored as training data and the data collection associated with that target view may be stopped (steps **212, 214).** This data generation process may be repeated for different target views, so that multiple sets of labelled image data of different view directions of a target, such as the heart, can be generated in an efficient way. Moreover, the data generation process may be repeated for a heterogenous group of patients of different size, age and gender. Thus, each set of labelled data may define different images that are captured by moving the probe relative to the target position.

During the data generation, the movement of the probe to different training positions may be a random movement within a predetermined area around the target position. Alternatively, the moment of the probe to different training positions within a predetermined area around the target position may be based on a predetermined function. Once one or more target views of a target are provided, the data collection module may autonomously generate training data, i.e. large sets of labelled data, for each respective target view that can be used to train a model, e.g. a deep neural network, for controlling the probe.

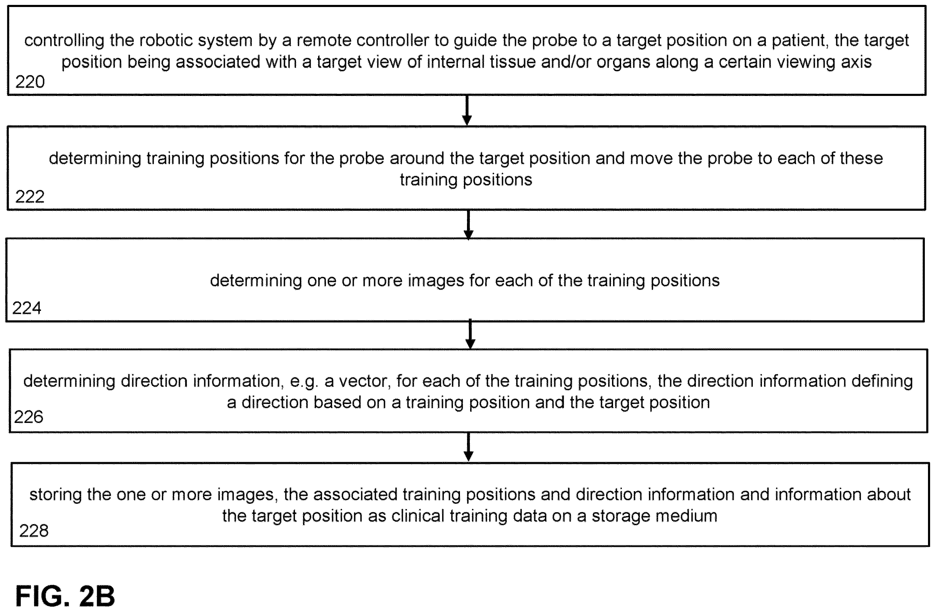

**Fig. 2B** depicts a flow chart of a method for generating clinical training data using a robotized echography system.

The process may include a first step **220** of controlling the robotic system using a remote controller to guide the probe to a target position on the patient. The target position may be associated with a target view of internal tissue and/or organs along a certain viewing axis. Then, in step **222** training positions, for example random training positions or training positions described by a certain function, for the probe may be determined around a target position. These training positions may be limited to a predetermined area around the target position and may include the target position. The probe may be moved to each of these training positions so that one or more images at each of the training positions can be determined by the probe (step **224**). Then, direction information for each of the training positions may be determined, wherein the direction information may define a direction, e.g. a vector, based on a training position and the target position (step **226**). Further, the one or more images, the associated training positions and direction information and information about the target position may be stored as clinical training data on a storage medium (step **228**).

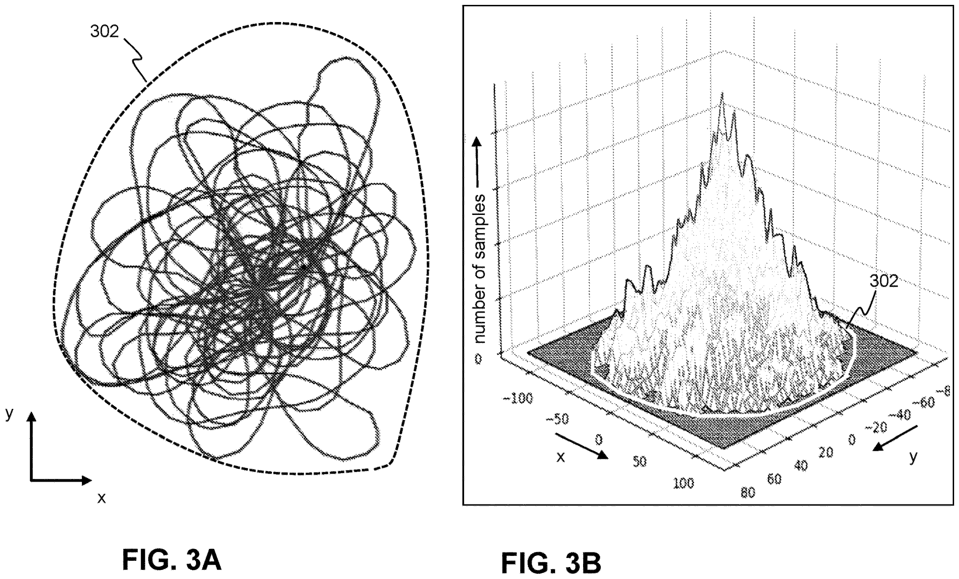

**Fig. 3A** and **3B** illustrate pictures associated with the generation of training data according to an embodiment of the invention. In particular, these figures illustrate an example of a path relative to a target position that the probe may follow based on position instructions by the data collector module during the data collection process. **Fig. 3A** depicts an example of a path wherein each subsequent position on the path may be obtained by changing the position of the probe (within certain limits) with a certain amount. The change in the position of the probe may include a translational change in the x,y coordinates of the probe and/or a rotational change in the angular coordinates of the probe. In some embodiments, the z-direction may be controlled by sensor feedback as explained above. The changes in the probe coordinates may define a direction (a heading) in which the probe moves. Further conditions may be added so that the path stays within a predetermined area **302,** which may be referred to as a data collection area. During the movement of the probe, one or more images may be captured at different training positions relative to the target positions, wherein the different training positions may include different translational and/or rotational (angular) positions.

Different algorithms may be used to control the generation of image data. For example, in an embodiment, the change in the probe direction may be based on the distance between the probe position and the target position. In another embodiment, a direction may be more preferential if it moves the probe closer to the target position. This preference may be dependent on the basis of a distance of the probe relative to the target position. For example, a preference to move the probe closer to the target position may be made stronger, the further the probe is removed from the target position. **Fig. 3B** illustrates that a probe may be configured to move withing a data collection area **302,** the closer the probe moves towards the optimal location within the data collection area (visualized by the position of the highest peak), the more images may be captured by the probe. The figure shows that the number of labelled images as a function of the position of the probe relative to the target view. The further away from the target position, the less labelled images are generated. Many different algorithms may be used to achieve this effect. This way, the number of labelled images generated at different positions around the target position may be controlled precisely.

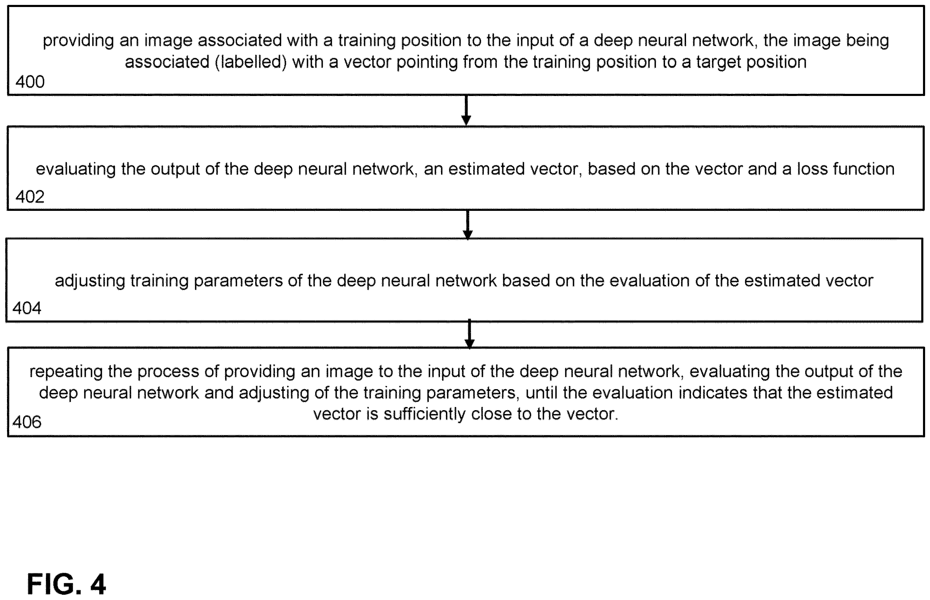

**Fig. 4** depicts a process for training a deep neural network based on labelled training data according to an embodiment of the invention. This process may be executed by the training module based on training data that may be generated by the data collection module as described with reference to **Fig. 1-3****.** As shown in **Fig. 4****,** in a first step **400** an image that is part of the clinical training data and that was taken by the probe at a training position may be provided to the input of a deep neural network implemented on the computer system of the robotized echography system. The image may be associated (labelled) with direction formation, e.g. a target vector pointing from the training position to a target position. Here, the position of the probe may include a translational part and an angular part and may be expressed based on a vector as described above with reference to **Fig. 1****.** Thus, a labelled image associated with a vector may be provided to the input of the deep neural network for training the deep neural network to generate an estimated vector that the robot controller can use to move the probe from a current position to a further position. The output of the neural network, i.e. the estimated vector, may be evaluated based on the vector and a loss function (step **402**). For example, a loss may be computed based on a normalized difference between the estimated vector and the vector associated with the image that was provided to the input of the deep neural network .

Based on the evaluation by the loss function, training parameters (e.g. weights of the activation functions of the neural network) may be adjusted using well-known algorithms that are based on backpropagation and gradient descent (step **404**). The process of providing a labelled image to the input of the deep neural network, evaluating the output of the deep neural network based on the loss function and adjusting the training parameters of the deep neural network may be repeated (step **406**) until the estimated vector is sufficiently close to the vector that is associated with the input image (step **408**). Here, the decision to stop the training process may be based on the computed loss being smaller than a certain threshold value and/or when the decreasing loss starts to increase due to overfitting. This process results in a trained neural network that is configured to receive an image from a probe at its input and to produce a vector that can be used to move the probe to a further probe position.

In some embodiments, the neural network system used by the robotized echography system may comprise a plurality of deep neural networks, wherein each neural network is trained for a particular target position. In that case, when the system is executed each of the trained neural networks used in finding a target position for an image in a predetermined viewing direction for a certain patient.

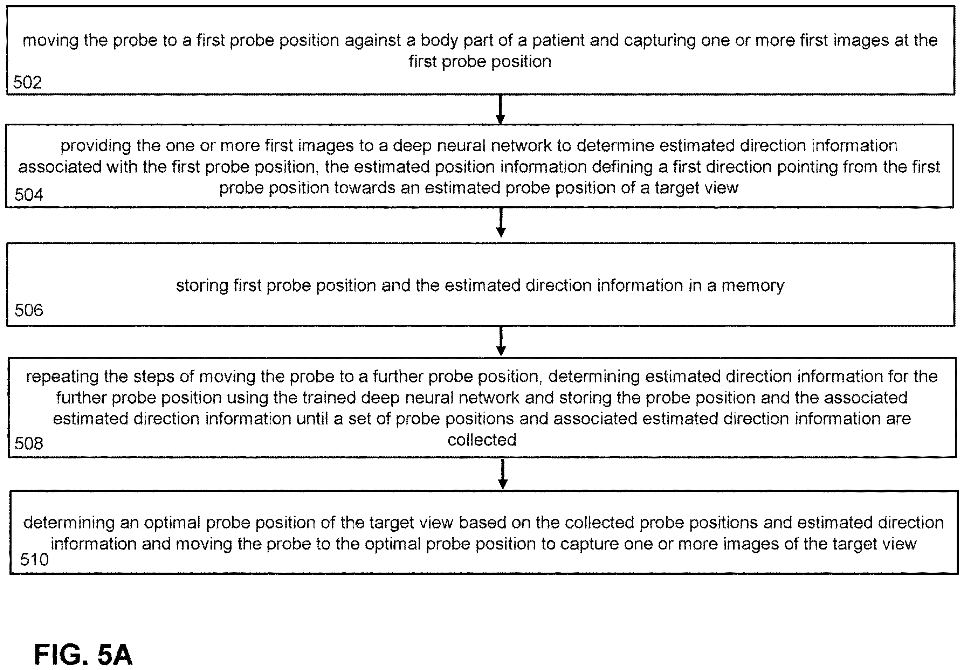

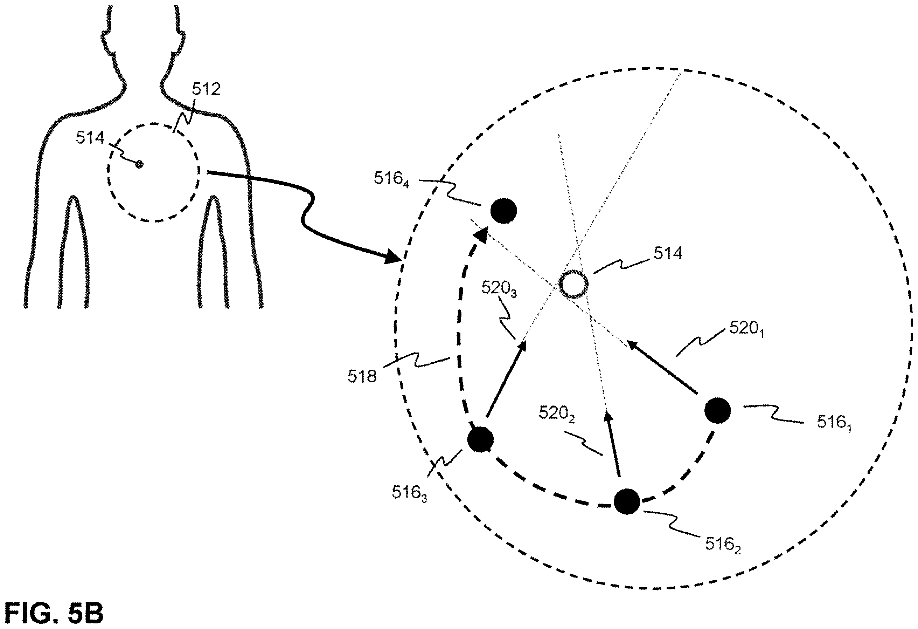

**Fig. 5A** and 5B depicts a process for executing a process of autonomously generating images of different target views of internal tissue and/or organs of part of a patient according to an embodiment of the invention. This process may be executed by the execution module as described with reference to **Fig. 1****.** The process may control the robotized echography system to autonomously capture multiple images of a target view, i.e. pictures associated with a viewing angle, of a body part of a patient that is positioned and fixated on a support structure, e.g. a bed. Each target view may be related to a predetermined view direction as known in the field of echography. For example, in echocardiography these predetermined view directions may include the parasternal view, the apical view, the subcostal view and the suprasternal view.

As shown in **Fig. 5A****,** the process may start with moving the probe to a first initial position against the body of the patient and capturing at the first probe position, one or more first images (step **502**). This step is schematically shown in **Fig. 5B** showing a patient for which images of a certain target view needs to be autonomously generated by the system. This target view is associated with an optimal probe position **514** which the system needs to determine based on the directional information that is determined by the trained neural network. To that end, the probe may be autonomously moved towards a first initial position **516₁** which is located in a certain area **512** around the unknown optimal probe position of the target view. A pressure sensor signal associated with the probe may be used by the system to provide a constant pressure against the body in a transversal direction (e.g. the z-direction), while the probe may be moved in translation directions (e.g. x and y direction) and rotational directions. Because the patient is in a fixed position relative to the probe, one or more initial positions may be defined. Such an initial position may be selected such that the probe is brought in contact with the patient within the area **512** that encompasses the optimal probe position **514** for generating images of the desired target view.

The one or more first images may be fed to the input of the trained neural network, which generates an output signal which includes first estimated direction information associated with the first probe position (step **504**). The first estimated direction information is schematically depicted in **Fig. 5B** by first vector **516₁**, which defines a first direction pointing from the first probe position towards an estimated probe position of the target view.

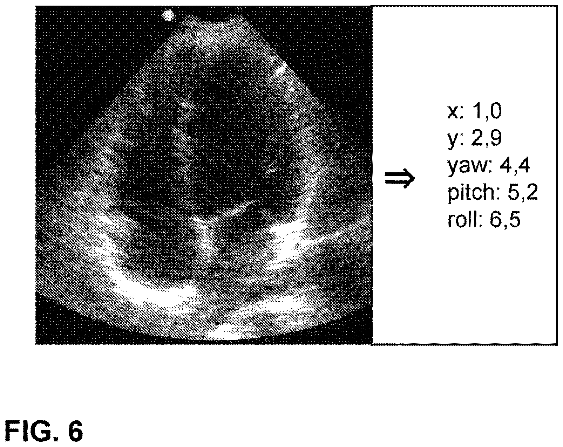

An example of an ultrasound image provided to the input of the deep neural network and an example of direction information that can be derived from the output of the deep neural network is illustrated in **Fig. 6****.** The estimated direction information may be in the form of a first vector or may include a first vector pointing from the first probe position to an estimated probe position of a target view. The first probe position and the first direction information may be stored in a memory (step **506**).

Then, based on the first direction information, the probe may be moved to a second probe position. While moving the probe to the second position, one or more second images may be captured and fed to the input of the trained neural network, which will produce second estimated direction information including a second vector pointing from the second probe position to an estimated proposition of the target view. Again, the second estimated probe position and second direction information may be stored and used to move the probe to a third probe position.

This process of moving the probe to a further probe position, determining further direction information including a further vector pointing from a current probe position towards a further estimated position associated with the target view and storing the probe position with the direction information may be repeated until a set of probe positions and associated estimated direction information are collected (step **508**). Based on the collected positions and direction information an optimal probe position of a target view may be determined and the probe may be moved to the optimal probe position to capture one or more images of the target view (step **510**).

This process is also shown in **Fig. 5B****,** showing the path **518** of a moving probe that includes probe locations **516₁₋₃** at which images are captured and direction information in the form of a vectors **520₁₋₃** which is generated by the trained neural network based on the captured images. One or more further probe locations, for example further probe location **516₄**, may be determined for determining further direction information. Each of the vectors points approximately towards an estimated probe location **514** of the target view. As shown in the figure, the probe locations and the vectors can be used to determine the optimal probe location for capturing images of the target view. Increasing the number of measured probe locations and associated vectors will increase the accuracy with which the probe position of the target view can be determined.

In some embodiments, additional information, in particular vital sign information, such as information from ECG and/or respiration signals, may be used to train the deep neural network.

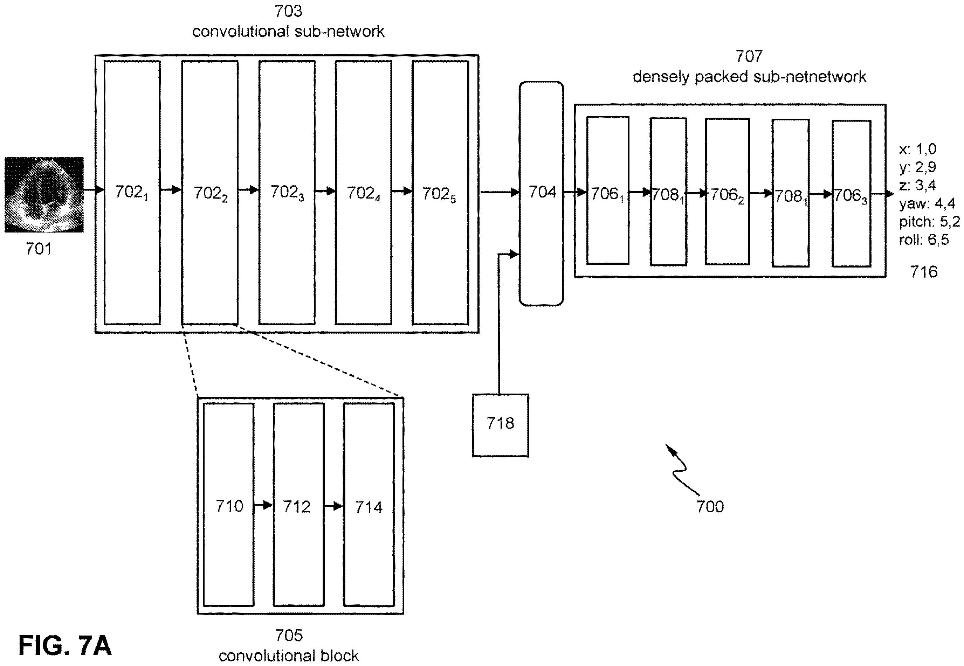

As described above, the deep neural network may be trained to predict a direction of the probe relative to a target position based on ultrasound images. As will be shown hereunder in greater detail, in some embodiments, the deep neural network may also be trained based on vital sign signals such as ECG and/or respiration signals. In an embodiment, one or more convolutional neural networks (CNNs) may be used CNNs are particular suitable for image processing. Various CNNs architectures may be selected to provide a network with an accuracy prediction. Exemplary embodiments of deep neural networks that may be used with the embodiments in this application are described hereunder in more detail with reference to **Fig. 7A-7C****.**

As shown in **Fig. 7A****,** the network **700** may include a number of sub-networks including a convolutional sub-network **703** and a densely packed sub-network **707.** The convolutional sub-network may comprise a plurality of convolutional blocks **702₁₋₅**, wherein the input of the convolutional sub-network is configured to receive one or more images **701.** Further, each convolutional block may comprise one or more convolutional layers **710,** which may be connected to one or more max pooling layers **712** and one or more dropout layers **714.** The output of the convolutional sub-network may be converted to a one-dimensional array by a flattening layer **704.** Before it is provided to the input of a densely connected sub-network **707** that comprises a plurality of densely connected layers **706₁₋₃**. In an embodiment, the densely connected layers may be interleaved with dropout layers **708₁₋₃**. In some embodiments, a sigmoid function may be used for the final activation layer **706₃**. In another embodiment, a ReLU activation function may be used for other (intermediary layers) convolutional and densely connected layers.

The input layers of the deep neural network may be configured to receive input data in the form of image data, typically pixelated 2D images representing slices of internal tissue and/or organs of a patient. In one embodiment, the input layer may be configured to receive a sequence of images in time, which may be used in a recurrent model. In another embodiment, the input layer may be configured to receive an individual image.

In an embodiment, in addition to image data, the deep neural network may also be trained on the basis of other data, for example vital sign signals such as ECG signals and/or respiration signals. This data may be added to the one-dimensional array produced by flattening layer **704.**

In some embodiment, the deep neural network cannot handle time dependent phenomena like movements of organs, like the hart. The heartbeat may cause parts of the hart to move in different directions. This may cause inaccuracies in the direction information as computed by the trained neural network. This problem may be addressed by adding additional information about the movement to the training data. Hence, one may extract features from an ECG signal that may help the deep neural network to interpreted an ultrasound image. One feature of the ECG signal is the phase of the heartbeat (which is a periodic signal). The phase of the heartbeat may be used as additional information that can be used for training the deep neural network in order to obtain a model that can more accurately determine the direction information based on an input image, e.g. a 2D slice, of the heart.

In a further embodiment, respiratory information may be used as additional information to train the deep neural network. The respiratory information may be beneficial for the deep neural network to recognize effects in images due to respiration. For example, if a patient inhales, the lungs will expand, which will be visible in the image as a "dark spot". This information can be added to the training data and used during training of the deep neural network as described with reference to **Fig. 4****.**

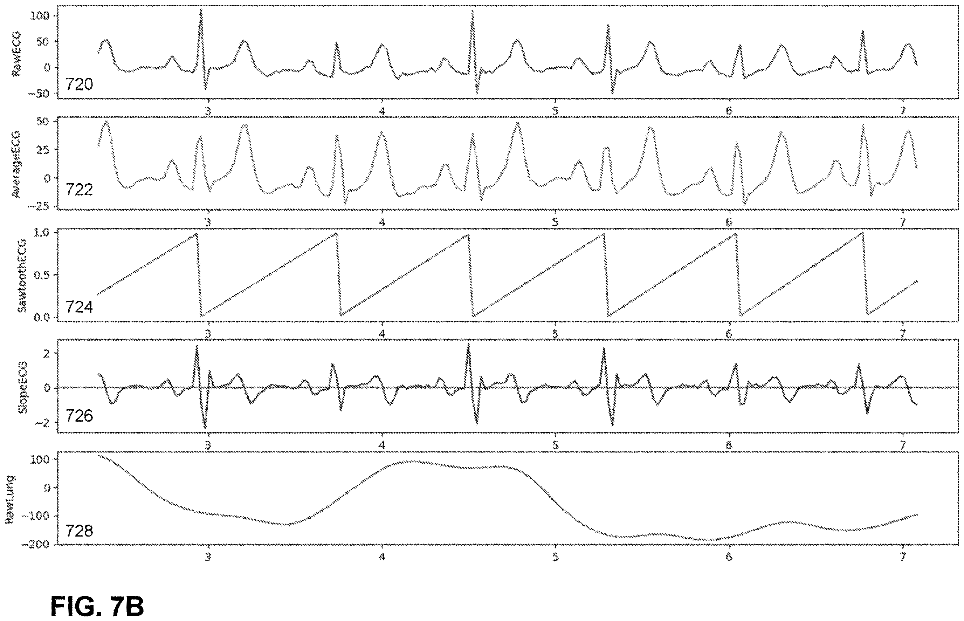

Examples of ECG and respiratory signals are shown in **Fig. 7B****.** An averaged ECG signal **722** and a derivative ECG signal **726** may be determined based on one or more raw ECG signals **720,** which are taken during the capturing of the images. These signals may be used to determine a heart phase signal **726** in the form of a saw tooth signal. In a similar, way a respiration signal **728** may be determined. Hence, during the generation of training data, images may be labelled with probe position, directional information and vital sign information such as the phase of the heartbeat and/or a measure of the respiration. The deep neural network may be trained based on this training data so that the deep neural network is more robust against effects from movement of tissue due to the heartbeat and/or respiration.

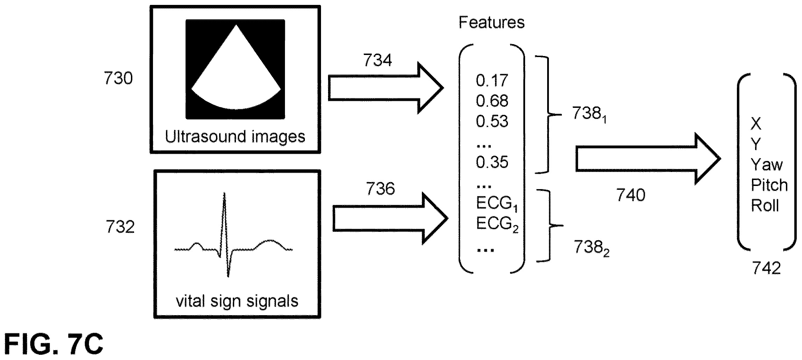

**Fig. 7C** depicts a high level scheme of the processing of ultrasound images by the trained neural network that is configured to autonomously control a robotized ultrasound imaging system. As shown in the figure, an ultrasound image **730** associated with a probe position may be processed by a trained deep neural network or part of a trained deep neural network (denoted by arrow **734**) into image features **738₁**. As shown in **Fig. 7A****,** the processing may be performed by a trained convolutional neural network or any other suitable deep neural network architecture. Similarly, in some embodiments, vital sign signals associated with the ultrasound image may be processed to extract vital sign features **738₂**, for example ECG and/or respiration features as illustrated in **Fig. 7B****.** Features may be extracted from vital sign signals using any type of feature extraction algorithm **736.** In some embodiments, the feature extraction algorithm may be trained neural networks that is trained for extracting useful features from the vital sign signal.

Subsequently, the features **738_{1,2}** may be processed **740** by a trained deep neural network, which is configured to generate direction information **742** in the form of a set of coordinates which may be regarded as a vector in the space in which the probe can move, wherein the vector has an origin at the position of the probe were the image is captured and wherein the coordinates provide a direction of the vector pointing towards an estimated probe position for the target view.

It is submitted that the figures are only non-limiting examples and many different variants exists without departing from the essence of the invention. For example, in an embodiment, the network architecture depicted in **Fig. 7A** or at least part thereof may be configured as a regression model. In that case, the number of convolution blocks in the convolutional sub-network may be five or more. The output of the model may include several parameters including translational parameters and angular parameters.

In further embodiment, the neural network be configured as a classification model. In that case, the translational and rotational axes may be divided in a predetermined number of bins. To do this, the network may classify the situation of each axis (x, y, z, yaw, pitch, roll, and optionally distance). The number of bins may have substantial impact on both the accuracy of the model and the training complexity. For example, two bins may imply a simple decision for one axis: "go left" or "go right", three bins may allow for a third choice, "no movement". Increasing the number of bins to five provides more granularity, allowing classes for "far left", "a little left", "no movement", "a little right", and "far right". The number of bins is a trade-off between ease of training (few bins), and precision of output (many bins).

Translation axes may need more granularity that the rotational axis. Therefore, the x,y,z-axis may for example be divided in five bins and the yaw, pitch, roll-axis may be divided into a lower number of bins, e.g. three bins. Further a binary cross-entropy may be used for a loss function and to track the total accuracy of the problem. For backpropagation a well-known stochastic gradient descent scheme may be used.

As already discussed with reference to **Fig. 7B****,** temporal data may be relevant for detecting the position of the probe, as attributes like heart-beats may contribute significantly to a prediction. Thus, in a further embodiment, the model may be configured as a recurrent convolution neural network (RCNN). Such model may be configured to process as time-series of images (e.g. a video clip) taking into account movements of the internal tissues and/or organs due to heart beat and/or respiration. A Long-Short Term Memory (LSTM) layer may be added to the RCNN to improve the accuracy of the system.

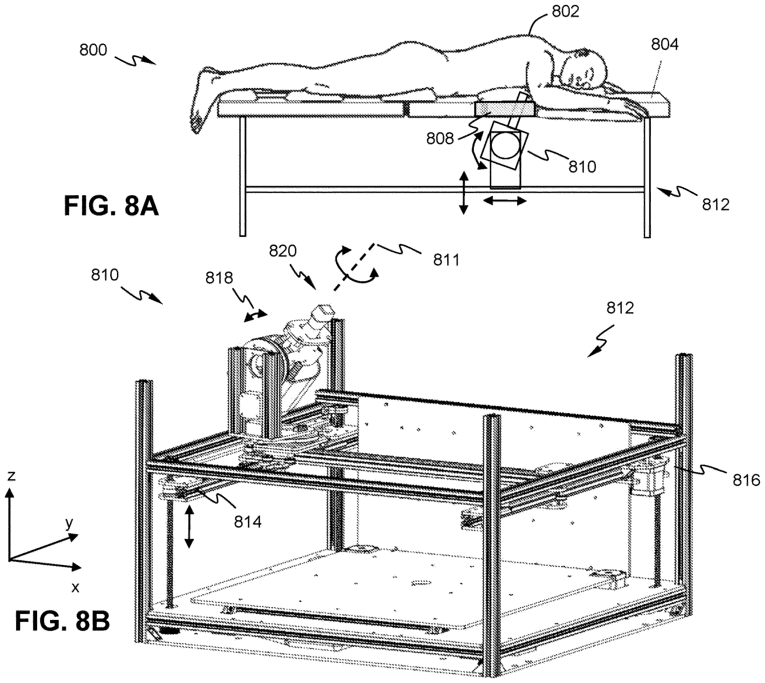

**Fig. 8A** and **8B** depicts an example of a robotized ultrasound echography system **800** according to an embodiment of the invention. As shown in **Fig. 8A****,** a patient **802** may be positioned and fixated on a support structure in prone position. The support structure may be implemented as a bed comprising a frame **812** and a horizontal support surface **804** comprising an opening **808** so that part of the body of the patient is exposed to a computer-controlled probe **810** which is configured to move both in translational and rotational directions.

**Fig. 8B** depicts the frame that includes the computer-controlled probe in more detail. As shown in the figure, a probe stage **810** may be mounted to a linear robot system, e.g. a cartesian coordinate robot, allowing translational movement of the stage in x,y,z directions. The movements of the probe stage may be realized by controlling electromotors and/or actuators **814,815,816** of the linear robot system. Translational movement of the probe stage may be realized by rotary motors through gears or pulleys. The probe stage may further include a gimbal structure which is designed to position the probe **820** in any desired angular position based on a suitable angular coordinate system: rotations in the xy plane, in the yz plane and in the plane perpendicular to the longitudinal axis **811** of the probe.

The probe should be positioned against the skin of the patient such that a force is exerted on the patient. Control of the force exerted onto the patient is important for obtaining good image quality. The force may be controllable between a range starting from a very light contact force up to a strong force, which is still within the comfortable range for the patient.
In order to allow the probe state to make the same movement as a human wrist, the yaw movement should be independent of the roll and pitch movement. Therefore, to achieve yaw, pitch and roll movements the yaw actuator is controlled as the last actuator (before contact with the patient) of the three series connected actuators. The yaw direction provides a movement effect that is similar to the human wrist.

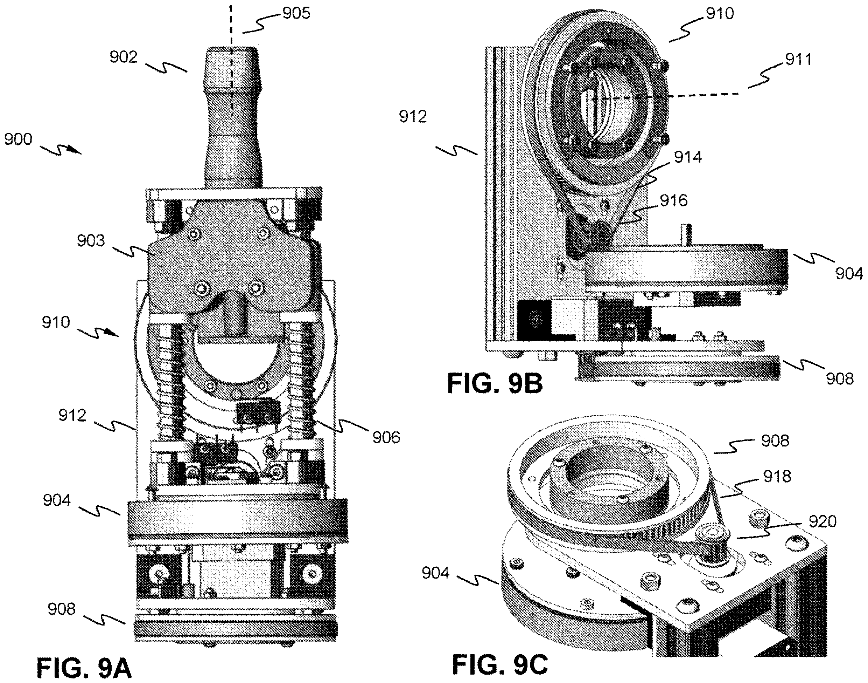

**Fig. 9A-9C** depicts various views of a rotatable probe stage for a robotized ultrasound echography system according to an embodiment of the invention. **Fig. 9A** represents a side view of the stage including a probe **902** that is mounted in a probe holder **903.** The probe may be any type of probe, including a commercially available manual echography probe. The probe holder fixates the probe to the probe stage. As shown in the figure, the probe holder is connected to a first rotatable disc **904** based on a fixture **906** comprising compressible springs of a known spring constant. The fixture allows movements of the probe relative to the probe stage in the direction parallel to the longitudinal axis **905** of the probe. Thus, when the probe is in flexible contact with the patient, the exerted force can be determined by determining the compression of the spring and computing the force based on the measured compression of the spring and the spring constant. The first rotatable disc **904** may be rotatably connected via an axle to a first drive pulley **908.** An output pulley **920** of a first motor **920** may be connected to the drive pulley via gearing, e.g. a belt-type gearing, as depicted in **Fig. 9C****.** This way, the first motor may control rotational movement of the probe around its longitudinal axis (which coincides with the rotational axis of first drive pulley **908**).

Further, the probe holder may be further rotatably connected to mounting structure **912** which is attached to linear robot as described with reference to **Fig. 8****.** As shown in **Fig. 9B** the rotatable connection rotatably connects the probe holder to the mounting structure. An output pulley **916** of a second motor may be connected to a second drive pulley **910** having a rotational axis **911** that is perpendicular to the rotational axis of first drive pulley **908.** This way, the second motor may control rotational movement of the probe around a rotational axis that is perpendicular to the longitudinal axis of the probe.

Thus, in contrast to known robotized echography systems, the echography system described with reference to **Fig. 8** and **9** requires a patient to be positioned on a support structure that comprises an opening that provides a robotized probe to access part of the body, e.g. the chest, of a patient that lies on the support structure in a prone position. This position is particularly advantageous for echographic imaging because organs, e.g. intestines or the heart, will not be blurred or at least less blurred by other tissue, e.g. lung tissue in case of the heart, as is the case if a patient is lying on its back. Thus, imaging a patient in the prone position will generate improved images, i.e. images with less distortions and/or interference due to lung tissue. For certain viewing directions, it will not be necessary to position the patient in different poses for imaging different views. Further, the opening in the support structure helps positioning and fixating a patient that needs to be scanned. The images of the patient in prone position that are generated by the data collector module are used for generating training data for training the model. Training the model based on the thus generated training data will result in a trained model of improved performance.

A further advantage of the system depicted in **Fig. 8** and **9** relates to the safety of the patient. In contrast to known robotized echocardiography systems, the patient is not positioned between the support structure, the bed, and the probe. Rather, the probe accesses the patient through an opening of the support structure so that the patient cannot get stuck between the probe and the bed, thus reducing the risk that malfunctioning of the robot may lead to situations in which the patient could be injured.

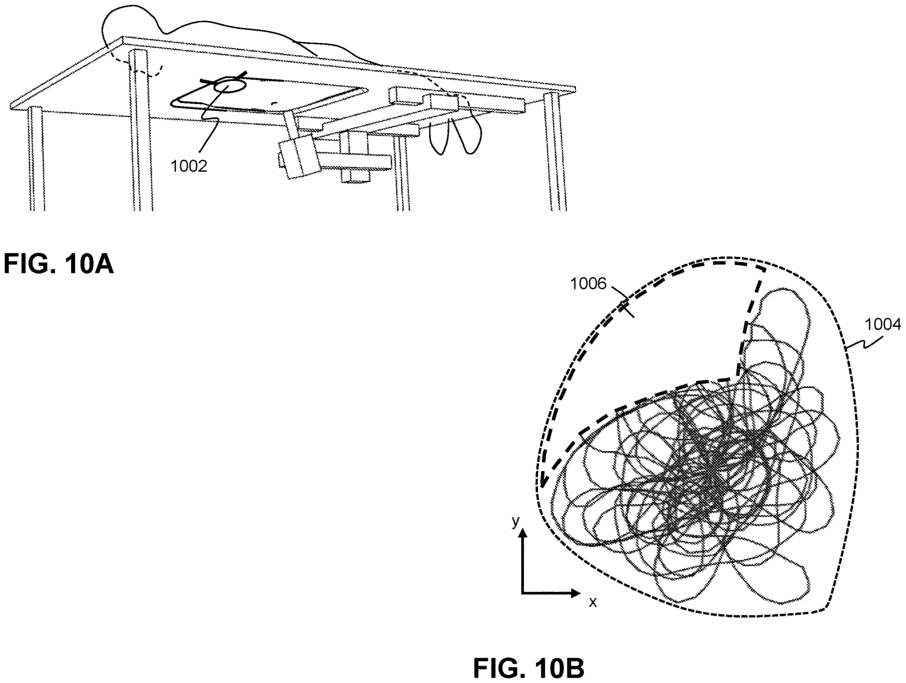

**Fig. 10A** and **10B** illustrates a robotized echography system according to an embodiment of the invention. The system may be similar to the one described with reference to **Fig. 1** including a support structure, e.g. a bed, for positioning a patient in a prone position so that the probe can access part of the body via the opening. In this embodiment, the data collection area **1004** in **Fig. 10B** in which the probe moves around a target spot, may include one or more marked areas **1006** in which the probe is not allowed. This way, sensitive areas, for example, areas including breast tissue can be avoided.

In an embodiment, such prohibited areas may be recognized by the system through the force feedback system. For example, when the probe moves around the target spot, a change in vertical z-axis of the probe larger than a certain threshold value may indicate that the probe moves into an area comprising breast tissue. In that case, the probe may move to a position in the data collection area that has a lower z-axis value. In another embodiment, a prohibited area may be marked by a mechanical element connected to the support structure. For example, in an embodiment, a breast may be placed against a suitable curved holder **1002** which may be mechanically mounted to the bed. The holder may prevent the probe from moving into a prohibited probe area.

These embodiments may be especially useful when imaging the apex of the heart, which is located at the left low border of the heart and located just under the breast. To avoid running over breast tissue with the probe, the target area may be approached by the probe from lateral and distal areas only, from where apex is expected, and not from above/from breast tissue. This area (distal or lateral from apex) may be found by using parasternal information, that shows the location of the apex too. This way, the search and data gathering may be limited to a part of the data collection area, instead of the full data collection area around target location.

It is submitted that the embodiments in this application are not limited to the embodiments illustrated in the figures. For example, while the figures illustrate a robotized echography system in which the patient is positioned in prone position on the support structure, in some embodiments, the system may scan body parts while the patient is arranged on the support structure in another pose, e.g. a patient on the back or the side are also, possible. Further, instead of arranging the probe under the support structure, in some embodiments, it is also possible that the robotic system comprising the robot is arranged over the patient. In that case, the support system does not need to have one or more openings or recesses for providing the probe access to one or more body parts of a patient.

The techniques of this disclosure may be implemented in a wide variety of devices or apparatuses. Various components, modules, or units are described in this disclosure to emphasize functional aspects of devices configured to perform the disclosed techniques, but do not necessarily require realization by different hardware units. Rather, as described above, various units may be combined in a codec hardware unit or provided by a collection of interoperative hardware units, including one or more processors as described above, in conjunction with suitable software and/or firmware.

The terminology used herein is for the purpose of describing particular embodiments only and is not intended to be limiting of the invention. As used herein, the singular forms "a," "an," and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

The description of the present invention has been presented for purposes of illustration and description, but is not intended to be exhaustive or limited to the invention in the form disclosed. Many modifications and variations will be apparent to those of ordinary skill in the art without departing from the scope of the invention. The embodiment was chosen and described in order to best explain the principles of the invention and the practical application, and to enable others of ordinary skill in the art to understand the invention for various embodiments with various modifications as are suited to the particular use contemplated.

## Claims

1. A robotized ultrasound imaging system comprising:
a robotic system (100) configured to position an ultrasound probe (106) against a body part of an object, e.g. a human object, positioned on a support structure (102), preferably a horizontal support structure, and to move the ultrasound probe over the body part; and,
a computer system (109) comprising:
a data generating module (116) configured to control the robotic system to generate clinical training data for a machine learning algorithm, the clinical training data comprising ultrasound images associated with
one or more target views of internal tissue and/or organs of the object, each target view being associated with a target position and orientation of the ultrasound probe, the training data further including for each target position a plurality of training positions and orientations of the probe relative to the target position and orientation and including direction information associated with the plurality of training positions and orientations, the direction information defining for each training position a direction, preferably a vector, pointing towards the target position;
a machine learning module (114) comprising a trained machine learning algorithm, which is trained based on the clinical training data, the machine learning module being configured to receive one or more ultrasound images (730) captured by the probe at a current probe position and orientation when moving over the surface of the body part of the object and to determine for the current probe position and orientation estimated direction information, preferably an estimated vector, defining a direction pointing from the current probe position and orientation towards an estimated probe position and orientation associated with a target view based on the one or more ultrasound images; and,
an execution module (120) configured to autonomously control the robotic system, the controlling including: moving the probe over the surface of the body part of the object to find an optimal probe position (514) and orientation for each of the one or more target views based on the estimated direction information generated by the trained machine learning algorithm for different probe positions (516) and orientations and to capture at the optimal probe position and orientation for each of the one or more target views, one or more ultrasound images of the internal tissue and/or organs.

2. Robotized imaging system according to claim 1, wherein the data generating module is further configured to:
determine a plurality of training positions and orientations for the probe within a predetermined data collection area defined around a target position and orientation;
move the probe to each of the training positions and orientations and capture one or more images for each of the training positions and orientations;
associate the one or more images associated with a training position and orientation with direction information, the direction information including coordinates of a vector pointing from the training position and orientation to the target position and orientation; and,
storing the one or more images and associated direction information as clinical training data on a storage medium.

3. Robotized imaging system according to claims 1 or 2, wherein the system comprises a training module (118) configured to:
provide an image associated with a training position and orientation to the input of the machine learning algorithm, the image being associated with direction information defining a direction, preferably a vector, pointing from the training position and orientation towards a target position and orientation;
receive estimated direction information, preferably an estimated vector, from the output of the machine learning algorithm and evaluating the estimated direction information based on the vector and a loss function;
adjust training parameters of the machine learning algorithm based on the evaluation of the estimated direction information; and,
repeat the process of providing an image to the input of the machine learning algorithm, evaluating the output of the deep neural network and adjusting of the training parameters, until the evaluation indicates that the estimated vector substantially ma the vector.

4. Robotized imaging system according to any of claims 1-3, wherein the execution module is configured to:
move the probe to a first probe position and orientation against a body part of a patient and capturing one or more first images at the first probe position and orientation;
provide the one or more first images to a deep neural network to determine estimated direction information associated with the first probe position and orientation, the estimated position information defining an estimated direction pointing from the first probe position and orientation towards an estimated probe position and orientation of the target view;
store first probe position and orientation and the estimate direction information in a memory;
repeat the steps of moving the probe to a further probe position and orientation, determining estimated direction information for the further probe position using the trained deep neural network and storing the probe position and the associated estimated direction information until a set of probe positions and associated estimated direction information are collected; and,
determine an optimal probe position and orientation of the target view based on the collected probe positions and orientations and estimated direction information and moving the probe to the optimal probe position and orientation to capture one or more images of the target view.

5. Robotized imaging system according to any of claims 1-4 wherein the machine learning algorithm is a deep neural network or a deep neural network system (700) comprising a plurality of concatenated deep neural networks, preferably the convolutional neural network or convolutional neural network system being configured as a regression algorithm or a classification algorithm.

6. Robotized imaging system according to claim 5 wherein the deep neural network or deep neural network system comprises one or more convolutional neural networks for extracting features from ultrasound images captured by the probe; and/or, wherein the deep neural network or deep neural network system comprises a network of densely connected layers for transforming features associated with an ultrasound image captured at a probe position into estimated direction information, wherein the estimated direction information defines a direction pointing from the probe position and orientation towards an estimated probe position and orientation of the target view.

7. Robotized imaging system according to any of claims 1-6 wherein the clinical training data further includes one or more features of one or more vital sign signals (732) associated with the one or more captured ultrasound images, preferably the one or more features including features of an ECG signal and/or features of a respiratory signal.

8. Robotized imaging system according to any of claims 1-7 wherein the support structure is configured to position the object in a prone position, the support structure comprising an opening or a recess exposing part of the object, preferably the chest of the object, to the probe, more preferably at least part of the robotic system being arranged under the support structure.

9. Robotized imaging system according to any of claims 1-7 wherein the robotic system includes a robot arm connected to the imaging probe for moving the probe in translational and rotational directions; or a linear robotic system, preferably arranged under the support structure, wherein the robotic system includes a linear robotic system configured to move a probe stage comprising the imaging probe in translational directions and wherein the probe stage is configured to move the imaging probe in angular directions.

10. Robotized imaging system according to claim 9 wherein the directional information is used by the robotic system to move the probe in one or more first directions, preferably the x,y directions, parallel to the plane of the support structure.

11. Robotized imaging system according to claims 9 or 10 wherein a sensor signal of a pressure sensor or a force sensor associated with the probe is used to move the probe in a second direction, preferably the z-direction, substantially perpendicular to the one or more first directions, preferably the sensor signal being used to press the probe with a constant force against the body part while the probe is moving in the one or more first directions.

12. Robotized imaging system according to any of claims 9-11 wherein the probe stage includes a probe holder for holding the imaging probe, the probe holder including the pressure or force sensor, preferably the pressure or force sensor including a spring structure, such that when the probe is pressed against the body part of the object, the one spring structure will be compressed, wherein the compression of the spring structure represents a value of the force with which the probe is pressed against the body part.

13. Robotized imaging system according to any of claims 1-12 wherein the direction information includes a set of translational coordinates for moving the imaging probe in a translational direction and a set of angular coordinates for moving the imaging probe in an angular direction.

14. Robotized imaging system according to any of claims 1-13 wherein the remote controller is shaped as a manual echography probe, the manual echography probe comprising one or more sensor, preferably one or more accelerometer sensors, one or more optical navigation sensors and/or inclination sensors for translating a position of the manual echography probe into positional information for controlling the robotic system to move the imaging probe to a position according to the positional information.

## Patentansprüche

1. Robotisiertes Ultraschall-Bildgebungssystem, das Folgendes aufweist:
ein Robotersystem (100), das konfiguriert ist, eine Ultraschallsonde (106) gegen ein Körperteil eines Objekts, z. B. eines menschlichen Objekts, das auf einer Stützstruktur (102), vorzugsweise einer horizontalen Stützstruktur, positioniert ist, zu positionieren und die Ultraschallsonde über das Körperteil zu bewegen; und
ein Computersystem (109), das Folgendes aufweist:
ein Datengenerierungsmodul (116), das konfiguriert ist, das Robotersystem zu steuern, um klinische Trainingsdaten für einen maschinellen Lernalgorithmus zu generieren, wobei die klinischen Trainingsdaten Ultraschallbilder aufweisen, die mit einer oder mehreren Zielansichten von internem Gewebe und/oder Organen des Objekts assoziiert sind, wobei jede Zielansicht mit einer Zielposition und -orientierung der Ultraschallsonde assoziiert ist, wobei die Trainingsdaten ferner für jede Zielposition eine Vielzahl von Trainingspositionen und -orientierungen der Sonde relativ zur Zielposition und -orientierung sowie Richtungsinformationen umfassen, die mit den mehreren Trainingspositionen und -orientierungen assoziiert sind, wobei die Richtungsinformationen für jede Trainingsposition eine Richtung definieren, vorzugsweise einen Vektor, der auf die Zielposition zeigt;
ein Modul für maschinelles Lernen (114), das einen trainierten maschinellen Lernalgorithmus aufweist, der auf den klinischen Trainingsdaten basiert, wobei das Modul für maschinelles Lernen konfiguriert ist, ein oder mehrere von der Sonde an einer aktuellen Sondenposition und -orientierung erfasste Ultraschallbilder (730) zu empfangen, wenn die Sonde über die Oberfläche des Körperteils des Objekts bewegt wird, und für die aktuelle Sondenposition und -orientierung geschätzte Richtungsinformationen zu bestimmen, vorzugsweise einen geschätzten Vektor, der eine Richtung definiert, die von der aktuellen Sondenposition und -orientierung auf eine geschätzte Sondenposition und -orientierung zeigt, die mit einer Zielansicht assoziiert ist, basierend auf den ein oder mehreren Ultraschallbildern; und
ein Ausführungsmodul (120), das konfiguriert ist, das Robotersystem autonom zu steuern, wobei die Steuerung Folgendes umfasst: die Sonde über die Oberfläche des Körperteils des Objekts zu bewegen, um eine optimale Sondenposition (514) und -orientierung für jede der einen oder mehreren Zielansichten basierend auf den von dem trainierten maschinellen Lernalgorithmus für verschiedene Sondenpositionen (516) und -orientierungen generierten geschätzten Richtungsinformationen zu finden und an der optimalen Sondenposition und -orientierung für jede der einen oder mehreren Zielansichten ein oder mehrere Ultraschallbilder des inneren Gewebes und/oder der Organe zu erfassen.

2. Robotisiertes Bildgebungssystem nach Anspruch 1, wobei das Datengenerierungsmodul ferner konfiguriert ist:
eine Vielzahl von Trainingspositionen und -orientierungen für die Sonde innerhalb eines vordefinierten Datenerfassungsbereichs um eine Zielposition und -orientierung herum zu bestimmen;
die Sonde zu jeder der Trainingspositionen und -orientierungen zu bewegen und ein oder mehrere Bilder für jede der Trainingspositionen und -orientierungen zu erfassen;
das eine oder die mehreren Bilder, die mit einer Trainingsposition und -orientierung assoziiert sind, mit Richtungsinformationen zu verknüpfen, wobei die Richtungsinformationen Koordinaten eines Vektors umfassen, der von der Trainingsposition und -orientierung zur Zielposition und -orientierung zeigt; und
das eine oder die mehreren Bilder und die zugehörigen Richtungsinformationen als klinische Trainingsdaten auf einem Speichermedium zu speichern.

3. Robotisiertes Bildgebungssystem nach Anspruch 1 oder 2, wobei das System ein Trainingsmodul (118) aufweist, das konfiguriert ist:
ein Bild, das mit einer Trainingsposition und -orientierung assoziiert ist, an den Eingang des maschinellen Lernalgorithmus zu liefern, wobei das Bild mit Richtungsinformationen assoziiert ist, die eine Richtung definieren, vorzugsweise einen Vektor, der von der Trainingsposition und -orientierung zur Zielposition und -orientierung zeigt;
geschätzte Richtungsinformationen, vorzugsweise einen geschätzten Vektor vom Ausgang des maschinellen Lernalgorithmus zu empfangen und die geschätzten Richtungsinformationen basierend auf dem Vektor und einer Verlustfunktion zu bewerten;
Trainingsparameter des maschinellen Lernalgorithmus basierend auf der Bewertung der geschätzten Richtungsinformationen anzupassen; und
den Prozess des Bereitstellens eines Bildes an den Eingang des maschinellen Lernalgorithmus, der Bewertung der Ausgabe des tiefen neuronalen Netzwerks und der Anpassung der Trainingsparameter zu wiederholen, bis die Bewertung anzeigt, dass der geschätzte Vektor im Wesentlichen mit dem Vektor übereinstimmt.

4. Robotisiertes Bildgebungssystem nach einem der Ansprüche 1 bis 3, wobei das Ausführungsmodul konfiguriert ist:
die Sonde zu einer ersten Sondenposition und -orientierung gegen ein Körperteil eines Patienten zu bewegen und ein oder mehrere erste Bilder an der ersten Sondenposition und -orientierung zu erfassen;
die ein oder mehreren ersten Bilder an ein tiefes neuronales Netzwerk zu liefern, um geschätzte Richtungsinformationen zu bestimmen, die mit der ersten Sondenposition und -orientierung assoziiert sind, wobei die geschätzten Positionsinformationen eine geschätzte Richtung definieren, die von der ersten Sondenposition und -orientierung auf eine geschätzte Sondenposition und -orientierung der Zielansicht zeigt;
die erste Sondenposition und -orientierung und die geschätzten Richtungsinformationen in einem Speicher zu speichern;
die Schritte des Bewegens der Sonde zu einer weiteren Sondenposition und -orientierung, des Bestimmens der geschätzten Richtungsinformationen für die weitere Sondenposition unter Verwendung des trainierten tiefen neuronalen Netzwerks und des Speicherns der Sondenposition und der zugehörigen geschätzten Richtungsinformationen zu wiederholen, bis eine Reihe von Sondenpositionen und zugehörigen geschätzten Richtungsinformationen gesammelt sind; und
eine optimale Sondenposition der Zielansicht basierend auf den gesammelten Sondenpositionen und geschätzten Richtungsinformationen zu bestimmen und die Sonde zur optimalen Sondenposition zu bewegen, um ein oder mehrere Bilder der Zielansicht zu erfassen.

5. Robotisiertes Bildgebungssystem nach einem der Ansprüche 1 bis 4, wobei der maschinelle Lernalgorithmus ein tiefes neuronales Netzwerk oder ein tiefes neuronales Netzwerksystem (700) umfasst, das eine Vielzahl von verketteten tiefen neuronalen Netzwerken aufweist, vorzugsweise das konvolutionsneuronale Netzwerk oder das konvolutionsneuronale Netzwerksystem, das als Regressionsalgorithmus oder Klassifikationsalgorithmus konfiguriert ist.

6. Robotisiertes Bildgebungssystem nach Anspruch 5, wobei das tiefe neuronale Netzwerk oder das tiefe neuronale Netzwerksystem ein oder mehrere konvolutionsneuronale Netzwerke zur Extraktion von Merkmalen aus von der Sonde erfassten Ultraschallbildern aufweist; und/oder wobei das tiefe neuronale Netzwerk oder das tiefe neuronale Netzwerksystem ein Netzwerk von dicht verbundenen Schichten aufweist, um Merkmale, die mit einem an einer Sondenposition erfassten Ultraschallbild assoziiert sind, in geschätzte Richtungsinformationen umzuwandeln, wobei die geschätzten Richtungsinformationen eine Richtung definieren, die von der Sondenposition und -orientierung auf eine geschätzte Sondenposition und -orientierung der Zielansicht zeigt.

7. Robotisiertes Bildgebungssystem nach einem der Ansprüche 1 bis 6, wobei die klinischen Trainingsdaten ferner ein oder mehrere Merkmale eines oder mehrerer Vitalzeichensignale (732) umfassen, die mit den erfassten Ultraschallbildern assoziiert sind, vorzugsweise umfassen die ein oder mehreren Merkmale Merkmale eines EKG-Signals und/oder Merkmale eines Atmungssignals.

8. Robotisiertes Bildgebungssystem nach einem der Ansprüche 1 bis 7, wobei die Stützstruktur konfiguriert ist, das Objekt in einer Bauchlage zu positionieren, wobei die Stützstruktur eine Öffnung oder eine Vertiefung aufweist, die einen Teil des Objekts, vorzugsweise die Brust des Objekts, der Sonde aussetzt, wobei vorzugsweise zumindest ein Teil des Robotersystems unter der Stützstruktur angeordnet ist.

9. Robotisiertes Bildgebungssystem nach einem der Ansprüche 1 bis 7, wobei das Robotersystem einen Roboterarm umfasst, der mit der Bildgebungssonde verbunden ist, um die Sonde in translatorischen und rotatorischen Richtungen zu bewegen; oder ein lineares Robotersystem, vorzugsweise unter der Stützstruktur angeordnet, wobei das Robotersystem ein lineares Robotersystem umfasst, das konfiguriert ist, eine Sondenstufe mit der Bildgebungssonde in translatorischen Richtungen zu bewegen, und wobei die Sondenstufe konfiguriert ist, die Bildgebungssonde in Winkeldirektionen zu bewegen.

10. Robotisiertes Bildgebungssystem nach Anspruch 9, wobei die Richtungsinformationen von dem Robotersystem verwendet werden, um die Sonde in einer oder mehreren ersten Richtungen zu bewegen, vorzugsweise in die xy-Richtungen parallel zur Ebene der Stützstruktur.

11. Robotisiertes Bildgebungssystem nach Anspruch 9 oder 10, wobei ein Sensorsignal eines Drucksensors oder eines Kraftsensors, der mit der Sonde verbunden ist, verwendet wird, um die Sonde in eine zweite Richtung zu bewegen, vorzugsweise in die z-Richtung, die im Wesentlichen senkrecht zu den einen oder mehreren ersten Richtungen verläuft, wobei vorzugsweise das Sensorsignal verwendet wird, um die Sonde mit einer konstanten Kraft gegen das Körperteil zu drücken, während die Sonde in den einen oder mehreren ersten Richtungen bewegt wird.

12. Robotisiertes Bildgebungssystem nach einem der Ansprüche 9 bis 11, wobei die Sondenstufe einen Sondenhalter umfasst, um die Bildgebungssonde zu halten, wobei der Sondenhalter den Druck- oder Kraftsensor umfasst, vorzugsweise der Druck- oder Kraftsensor eine Federstruktur umfasst, so dass, wenn die Sonde gegen den Körperteil des Objekts gedrückt wird, die Federstruktur komprimiert wird, wobei die Kompression der Federstruktur einen Wert der Kraft darstellt, mit der die Sonde gegen den Körperteil gedrückt wird.

13. Robotisiertes Bildgebungssystem nach einem der Ansprüche 1 bis 12, wobei die Richtungsinformationen ein Satz von translatorischen Koordinaten zum Bewegen der Bildgebungssonde in einer translatorischen Richtung und ein Satz von Winkelkoordinaten zum Bewegen der Bildgebungssonde in einer Winkeldirektion umfassen.

14. Robotisiertes Bildgebungssystem nach einem der Ansprüche 1 bis 13, wobei der Fernsteuerung als manuelle Echographie-Sonde geformt ist, wobei die manuelle Echographie-Sonde einen oder mehrere Sensoren umfasst, vorzugsweise einen oder mehrere Beschleunigungssensoren, einen oder mehrere optische Navigationssensoren und/oder Neigungssensoren zur Übersetzung einer Position der manuellen Echographie-Sonde in Positionsinformationen zur Steuerung des Robotersystems, um die Bildgebungssonde entsprechend den Positionsinformationen zu einer Position zu bewegen.

## Revendications

1. Système d'imagerie ultrasonore robotisé comprenant :
un système robotique (100) configuré pour positionner une sonde ultrasonore (106) contre une partie de corps d'un objet, par exemple un objet humain, positionné sur une structure de support (102), de préférence une structure de support horizontale, et pour déplacer la sonde ultrasonore au-dessus de la partie de corps ; et,
un système informatique (109) comprenant :
un module de génération de données (116) configuré pour commander le système robotique pour générer des données d'entraînement cliniques pour un algorithme d'apprentissage machine, les données d'entraînement cliniques comprenant des images ultrasonores associées à une ou plusieurs vues cibles d'un tissu interne et/ou d'organes de l'objet, chaque vue cible étant associée à une position et orientation cible de la sonde ultrasonore, les données d'entraînement comportant en outre pour chaque position cible une pluralité de positions et d'orientations d'entraînement de la sonde par rapport aux position et orientation cibles et comportant des informations de direction associées à la pluralité de positions et d'orientations d'entraînement, les informations de direction définissant pour chaque position d'entraînement une direction, de préférence un vecteur, pointant vers la position cible ;
un module d'apprentissage machine (114) comprenant un algorithme d'apprentissage machine entraîné, qui est entraîné sur la base des données d'entraînement cliniques, le module d'apprentissage machine étant configuré pour recevoir une ou plusieurs images ultrasonores (730) capturées par la sonde au niveau d'une position et orientation de sonde actuelle lorsqu'elle se déplace au-dessus de la surface de la partie de corps de l'objet et pour déterminer pour les position et orientation de sonde actuelles des informations de direction estimée, de préférence un vecteur estimé, définissant une direction pointant à partir des position et orientation de sonde actuelle en direction d'une position et orientation de sonde estimée associée à une vue cible sur la base des une ou plusieurs images ultrasonores ; et,
un module d'exécution (120) configuré pour commander de manière autonome le système robotique, la commande comportant : le déplacement de la sonde au-dessus de la surface de la partie de corps de l'objet pour trouver une position (514) et orientation de sonde optimale pour chacune des une ou plusieurs vues cibles sur la base des informations de direction estimée générées par l'algorithme d'apprentissage machine entraîné pour différentes positions et orientations de sonde (516) et pour capturer au niveau de la position et orientation de sonde optimale pour chacune des une ou plusieurs vues cibles une ou plusieurs images ultrasonores des tissu interne et/ou organes.

2. Système d'imagerie robotisé selon la revendication 1, dans lequel le module de génération de données est en outre configuré pour :
déterminer une pluralité de positions et d'orientations d'entraînement pour la sonde dans une zone de collecte de données prédéterminée définie autour d'une position et orientation cible ;
déplacer la sonde vers chacune des positions et orientations d'entraînement et capturer une ou plusieurs images pour chacune des positions et orientations d'entraînement ;
associer les une ou plusieurs images associées à une position et orientation d'entraînement à des informations de direction, les informations de direction comportant les coordonnées d'un vecteur pointant à partir de la position et de l'orientation d'entraînement vers les position et orientation cibles ; et,
stocker les une ou plusieurs images et informations de direction associées comme données d'entraînement cliniques sur un support de stockage.

3. Système d'imagerie robotisé selon les revendications 1 ou 2, dans lequel le système comprend un module d'entraînement (118) configuré pour :
fournir une image associée à une position et orientation d'entraînement à l'entrée de l'algorithme d'apprentissage machine, l'image étant associée aux informations de direction définissant une direction, de préférence un vecteur, pointant à partir de la position et orientation d'entraînement en direction d'une position et orientation cible ;
recevoir des informations de direction estimée, de préférence un vecteur estimé, de la sortie de l'algorithme d'apprentissage machine et évaluer les informations de direction estimée sur la base du vecteur et d'une fonction de perte ;
ajuster les paramètres d'entraînement de l'algorithme d'apprentissage machine sur la base de l'évaluation des informations de direction estimée ; et,
répéter le processus de fourniture d'une image à l'entrée de l'algorithme d'apprentissage machine, d'évaluation de la sortie du réseau neuronal profond et d'ajustement des paramètres d'entraînement, jusqu'à ce que l'évaluation indique que le vecteur estimé sensiblement ma le vecteur.

4. Système d'imagerie robotisé selon l'une quelconque des revendications 1-3, dans lequel le module d'exécution est configuré pour :
déplacer la sonde vers une première position et orientation de sonde contre une partie de corps d'un patient et capturer une ou plusieurs premières images au niveau de la première position et orientation de sonde ;
fournir les une ou plusieurs premières images à un réseau neuronal profond pour déterminer des informations de direction estimée associées à la première position et orientation de sonde, les informations de position estimée définissant une direction estimée pointant à partir de la première position et orientation de sonde en direction d'une position et orientation de sonde estimée de la vue cible ;
stocker la première position et orientation de sonde et les informations de direction estimée dans une mémoire ;
répéter les étapes de déplacement de la sonde vers une autre position et orientation de sonde, déterminer les informations de direction estimée pour l'autre position de sonde au moyen du réseau neuronal profond entraîné et stocker la position de sonde et les informations de direction estimée associées jusqu'à ce qu'un ensemble de positions de sonde et d'informations de direction estimée associées soient collectées ; et,
déterminer une position et orientation de sonde optimale de la vue cible sur la base des positions et orientations de sonde et des informations de direction estimée collectées et déplacement de la sonde vers la position et l'orientation de sonde optimale pour capturer une ou plusieurs images de la vue cible.

5. Système d'imagerie robotisé selon l'une quelconque des revendications 1-4 dans lequel l'algorithme d'apprentissage machine est un réseau neuronal profond ou un système de réseau neuronal profond (700) comprenant une pluralité de réseaux neuronaux profonds concaténés, de préférence le réseau neuronal convolutif ou système de réseau neuronal convolutif étant configuré comme un algorithme de régression ou un algorithme de classification.

6. Système d'imagerie robotisé selon la revendication 5 dans lequel le réseau neuronal profond ou système de réseau neuronal profond comprend un ou plusieurs réseaux neuronaux convolutifs pour extraire des caractéristiques d'images ultrasonores capturées par la sonde ; et/ou dans lequel le réseau neuronal profond ou système de réseau neuronal profond comprend un réseau de couches reliées de manière dense pour transformer des caractéristiques associées à une image ultrasonore capturée au niveau d'une position de sonde en informations de direction estimée, dans lequel les informations de direction estimée définissent une direction pointant à partir de la position et de l'orientation de sonde en direction d'une position et orientation de sonde estimée de la vue cible.

7. Système d'imagerie robotisé selon l'une quelconque des revendications 1-6 dans lequel les données d'entraînement cliniques comportent en outre une ou plusieurs caractéristiques d'un ou de plusieurs signaux de signe vital (732) associées aux une ou plusieurs images ultrasonores capturées, de préférence les unes ou plusieurs caractéristiques comportant les caractéristiques d'un signal ECG et/ou caractéristiques d'un signal respiratoire.

8. Système d'imagerie robotisé selon l'une quelconque des revendications 1-7 dans lequel la structure de support est configurée pour positionner l'objet en décubitus ventral, la structure de support comprenant une ouverture ou un évidement exposant une partie de l'objet, de préférence la poitrine de l'objet, à la sonde, de manière davantage préférée au moins une partie du système robotique étant agencée sous la structure de support.

9. Système d'imagerie robotisé selon l'une quelconque des revendications 1-7 dans lequel le système robotique comporte un bras de robot relié à la sonde d'imagerie pour déplacer la sonde dans des directions de translation et de rotation ; ou un système robotique linéaire, de préférence agencé sous la structure de support, dans lequel le système robotique comporte un système robotique linéaire configuré pour déplacer un étage de sonde comprenant la sonde d'imagerie dans des directions de translation et dans lequel l'étage de sonde est configuré pour déplacer la sonde d'imagerie dans des directions angulaires.

10. Système d'imagerie robotisé selon la revendication 9 dans lequel les informations de direction sont utilisées par le système robotique pour déplacer la sonde dans une ou plusieurs premières directions, de préférence les directions x, y, parallèlement au plan de la structure de support.

11. Système d'imagerie robotisé selon les revendications 9 ou 10 dans lequel un signal de capteur d'un capteur de pression ou d'un capteur de force associé à la sonde est utilisé pour déplacer la sonde dans une deuxième direction, de préférence la direction z, sensiblement perpendiculaire aux une ou plusieurs premières directions, de préférence le signal de capteur étant utilisé pour presser la sonde avec une force constante contre la partie de corps pendant que la sonde se déplace dans les une ou plusieurs premières directions.

12. Système d'imagerie robotisé selon l'une quelconque des revendications 9-11 dans lequel l'étage de sonde comporte un porte-sonde pour porter la sonde d'imagerie, le porte-sonde comportant le capteur de pression ou de force, de préférence le capteur de pression ou de force comportant une structure de ressort, de telle sorte que lorsque la sonde est pressée contre la partie de corps de l'objet, la une structure de ressort sera compressée, dans lequel la compression de la structure de ressort représente une valeur de la force avec laquelle la sonde est pressée contre la partie de corps.

13. Système d'imagerie robotisé selon l'une quelconque des revendications 1-12 dans lequel les informations de direction comportent un ensemble de coordonnées de translation pour déplacer la sonde d'imagerie dans une direction de translation et un ensemble de coordonnées angulaires pour déplacer la sonde d'imagerie dans une direction angulaire.

14. Système d'imagerie robotisé selon l'une quelconque des revendications 1-13 dans lequel la télécommande est formée comme une sonde d'échographie manuelle, la sonde d'échographie manuelle comprenant un ou plusieurs capteurs, de préférence un ou plusieurs capteurs accéléromètres, un ou plusieurs capteurs de navigation optiques et/ou capteurs d'inclinaison pour translater une position de la sonde d'échographie manuelle en informations de position pour commander le système robotique pour déplacer la sonde d'imagerie vers une position en fonction des informations de position.
